(19) Europäisches Patentamt
European Patent Office
Office européen des brevets

(11) EP 4 162 922 A1

(12) EUROPEAN PATENT APPLICATION
published in accordance with Art. 153(4) EPC

(43) Date of publication:
**12.04.2023 Bulletin 2023/15**

(21) Application number: **21822132.3**

(22) Date of filing: **03.06.2021**

(51) International Patent Classification (IPC):
***A61K 8/34*** (2006.01) ***A61K 8/31*** (2006.01)
***A61K 8/49*** (2006.01) ***A61K 8/81*** (2006.01)
***A61K 8/891*** (2006.01) ***A61K 8/898*** (2006.01)
***A61Q 1/02*** (2006.01) ***A61Q 3/00*** (2006.01)
***A61Q 5/00*** (2006.01)

(52) Cooperative Patent Classification (CPC):
**A61K 8/31; A61K 8/34; A61K 8/49; A61K 8/81; A61K 8/891; A61K 8/898; A61Q 1/02; A61Q 3/00; A61Q 5/00**

(86) International application number:
**PCT/JP2021/021213**

(87) International publication number:
**WO 2021/251268 (16.12.2021 Gazette 2021/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.06.2020 JP 2020099623**

(71) Applicant: **Kao Corporation**
**Chuo-ku**
**Tokyo 103-8210 (JP)**

(72) Inventor: **FUKUDA, Teruyuki**
**Wakayama-shi, Wakayama 640-8580 (JP)**

(74) Representative: **Hoffmann Eitle**
**Patent- und Rechtsanwälte PartmbB**
**Arabellastraße 30**
**81925 München (DE)**

# (54) COSMETIC COMPOSITION

(57) The invention relates to: a cosmetic composition containing a solvent A, a solvent B, a polymer C and a colorant, wherein the solvent Ais at least one selected from the group consisting of ethanol, n-propanol and isopropanol, the boiling point of the solvent B is 150°C or higher, and the distance Ra of the Hansen solubility parameter of the solvent B to water is 40 or more, the solvent B is compatible with the solvent A, and the polymer C is soluble in the solvent A and insoluble in the solvent B; a makeup method using the cosmetic composition; and a cosmetic coating film formed from the cosmetic composition.

EP 4 162 922 A1

## Description

### FIELD OF THE INVENTION

The present invention relates to a cosmetic composition, a make-up method using the cosmetic composition, and a cosmetic coating film formed from the cosmetic composition.

### BACKGROUND OF THE INVENTION

Conventionally, in order to cover spots, dullness and pores of skin or to improve the brightness of coating finish, makeup cosmetics have been blended with an inorganic pigment with a high concealing power, such as titanium oxide, zinc oxide, and iron oxide.

For example, JP 2016-121137 A (PTL 1) describes a cosmetic containing titanium oxide having an average particle size of 0.2 pm or more and resin fine particles having an average particle size of 0.01 to 100 pm for the purpose of providing a cosmetic capable of giving high whiteness or concealing power.

In addition, JP 2015-520120 A (PTL 2) describes a cosmetic composition containing a plate type filler having predetermined refractive index and particle size, a silicone elastomer, and a filler having an oil absorption capacity of 1 mL/g or more in a physiologically acceptable medium for the purpose of long lasting an effect for hiding the skin imperfections or the like.

### SUMMARY OF THE INVENTION

The present invention relates to a cosmetic composition containing a solvent A, a solvent B, a polymer C and a colorant, wherein:

the solvent A is at least one selected from the group consisting of ethanol, n-propanol and isopropanol,
the boiling point of the solvent B is 150°C or higher, and the distance Ra of the Hansen solubility parameter of the solvent B to water as expressed by the following equation (1) is 40 or more,
the solvent B is compatible with the solvent A, and the polymer C is soluble in the solvent A and insoluble in the solvent B.

$$Ra=(4\times\Delta D^2+\Delta P^2+\Delta H^2)^{0.5} \qquad (1)$$

wherein,

$\Delta D$ is a difference of dispersing component in the Hansen solubility parameter between the solvent B and water,
$\Delta P$ is a difference of polar component in the Hansen solubility parameter between the solvent B and water, and
$\Delta H$ is a difference of hydrogen-bonding component in the Hansen solubility parameter between the solvent B and water.

### DETAILED DESCRIPTION OF THE INVENTION

Here, base makeup cosmetic materials such as foundations and concealers that are especially for skin color control are desired to have high color developing performance capable of concealing spots, bruises and pores of skin and capable of coloring skin in the color intrinsic to the colorant blended in the cosmetic materials.

Point makeup cosmetic materials such as eyeshadows, cheek rouges and nail enamels that are applied to parts of face or nails to partly apply color to highlight them are used for the purpose of applying shadows to give a stereoscopic effect or for the purpose of applying colors for partially emphasizing, and are therefore required to have high color developing performance capable of concealing the color intrinsic to skin and nails and capable of coloring skin and nails in the color intrinsic to the colorant blended in the cosmetic materials.

Further, temporary hair colors such as hair mascaras are preferred as those that give less damage to hair and can pleasantly enjoy hair coloring, and are used for the purpose of imparting bright color to hair especially for improving fashionability. However, temporary hair colors are to color hair by forming a coloring film on hair, and are desired to have high color developing performance capable of concealing the color intrinsic to hair and capable of coloring hair in the color intrinsic to the colorant blended in the temporary hair colors.

The techniques of PTLs 1 and 2 are to express a hiding power by blending with an inorganic pigment having a high refractive index. However, recently, with respect to zinc oxide and titanium oxide, the restrictions on use are

becoming severe due to various laws and regulations.

For that reason, the development of cosmetic compositions capable of forming cosmetic coating films excellent in concealing performance and coloring performance without using an inorganic pigment is desired.

Further, some temporary hair colors are used in a use form where hair is dyed in a mesh style, and therefore, a simple makeup method capable of coloring only a desired part of hair is desired.

The present invention relates to a cosmetic composition capable of forming a cosmetic coating film excellent in concealing performance and coloring performance without using an inorganic pigment, to a makeup method using the cosmetic composition, and to a cosmetic coating film formed from the cosmetic composition.

The present inventor has found that a cosmetic composition containing two kinds of solvents, a polymer and a colorant, in which the first solvent of the two kinds of solvents is at least one selected from the group consisting of ethanol, n-propanol and isopropanol, the boiling point and the distance of the Hansen solubility parameter to water of the second solvent each are a specific value or more, and the compatibility of the two kinds of solvents and the solubility of the polymer in the respective solvents are in a specific relationship, can form a cosmetic coating film excellent in concealing performance and coloring performance without using an inorganic pigment.

Specifically, the present invention relates to the following [1] to [3].

[1] A cosmetic composition containing a solvent A, a solvent B, a polymer C and a colorant, wherein:

the solvent A is at least one selected from the group consisting of ethanol, n-propanol and isopropanol,
the boiling point of the solvent B is 150°C or higher, and the distance Ra of the Hansen solubility parameter of the solvent B to water as expressed by the following equation (1) is 40 or more,
the solvent B is compatible with the solvent A, and the polymer C is soluble in the solvent A and insoluble in the solvent B.

$$Ra=(4\times\Delta D^2+\Delta P^2+\Delta H^2)^{0.5} \quad (1)$$

wherein,

$\Delta D$ is a difference of dispersing component in the Hansen solubility parameter between the solvent B and water,
$\Delta P$ is a difference of polar component in the Hansen solubility parameter between the solvent B and water, and
$\Delta H$ is a difference of hydrogen-bonding component in the Hansen solubility parameter between the solvent B and water.

[2] A makeup method using the cosmetic composition of the above [1], which includes:

Step 1: a step of applying the cosmetic composition to skin, hair or nails, and
Step 2: a step of applying drops of a water-containing liquid E to the cosmetic composition applied on skin, hair or nails.

[3] A cosmetic coating film formed from the cosmetic composition of the above [1].

In accordance with the present invention, it is possible to provide a cosmetic composition capable of forming a cosmetic coating film excellent in concealing performance and coloring performance without using an inorganic pigment, a makeup method using the cosmetic composition, and a cosmetic coating film formed from the cosmetic composition.

[Cosmetic Composition]

The cosmetic composition of the present invention contains a solvent A, a solvent B, a polymer C and a colorant, wherein the solvent Ais at least one selected from the group consisting of ethanol, n-propanol and isopropanol, the boiling point of the solvent B is 150°C or higher, and the distance Ra of the Hansen solubility parameter of the solvent B to water as expressed by the following equation (1) is 40 or more, the solvent B is compatible with the solvent A, and the polymer C is soluble in the solvent A and insoluble in the solvent B.

$$Ra=(4\times\Delta D^2+\Delta P^2+\Delta H^2)^{0.5} \quad (1)$$

wherein,

ΔD is a difference of dispersing component in the Hansen solubility parameter between the solvent B and water,
ΔP is a difference of polar component in the Hansen solubility parameter between the solvent B and water, and
ΔH is a difference of hydrogen-bonding component in the Hansen solubility parameter between the solvent B and water.

In the present invention, the wording "compatible" refers to a phenomenon in which in a mixed system containing the solvent A and the solvent B, the solvent A and the solvent B are mutually dissolved. The case where when the solvent A and the solvent B are mixed and allowed to stand, they are not separated in multiple phases, or the case where when the solvent A and the solvent B are mixed and subjected to a stirring operation, no phase separation is caused, so that they do not become cloudy, is judged such that the solvent A and the solvent B are in a compatibilized state with each other.
The polymer C is one which is soluble in the solvent A but insoluble in the solvent B and is dissolved in the cosmetic composition.
In the present invention, the wording "the polymer C is soluble in the solvent A" means that the dissolved amount when the polymer C after drying at 105°C for 2 hours and reaching a constant weight is dissolved in 100 g of the solvent A at 25°C is 5 g or more. The aforementioned dissolved amount of the polymer C in the solvent A is, from the viewpoint of improving concealing performance and coloring performance, preferably 10 g or more.
In the present invention, the wording "the polymer C is insoluble in the solvent B" means that the dissolved amount when the polymer C after drying at 105°C for 2 hours and reaching a constant weight is dissolved in 100 g of the solvent B at 25°C until it is saturated is less than 5 g. The foregoing dissolved amount of the polymer C in the solvent B is, from the viewpoint of improving concealing performance and coloring performance, preferably less than 2 g.
The judgement regarding "compatible" or "soluble" is performed at 25°C.
In the present invention, the "Hansen solubility parameter" is expressed by dividing the solubility parameter (SP value) introduced by Hildebrand into three components (dispersing component D, polar component P, and hydrogen-binding component H). The D, P, and H of the respective solvents are described in detail in "HANSEN SOLUBILITY PARAMETERS" A User's Handbook Second Edition. In addition, the HSP values regarding a lot of solvents and resins are also described in Wesley L. Archer, "Industrial Solvents Handbook" and the like.
The D, P, and H of the respective solvents can also be determined using a software HSPiP of Charles Hansen Consulting, Inc. (Horsholm, Denmark, hansen-solubility. com).
In the present invention, with respect to solvents registered in the database of HSPiP Version 4.1.03 (see the literatures of various HSP's), the values are adopted, and with respect to solvents not registered in the database, values estimated from the aforementioned HSPiP are adopted.
In the present invention, the unit of the "Hansen solubility parameter" is "$(MPa)^{0.5}$".
In accordance with the present invention, it is possible to form a cosmetic coating film excellent in concealing performance and coloring performance even when an inorganic pigment is not used. Although the reason for this is not elucidated yet, the following may be considered.
The cosmetic composition of the present invention contains the solvent A of at least one selected from the group consisting of ethanol, n-propanol and isopropanol, the solvent B of which the boiling point is 150°C or higher and of which the distance Ra of the Hansen solubility parameter to water as expressed by the above-mentioned equation (1) is 40 or more, the polymer C which is soluble in the solvent A and insoluble in the solvent B, and a colorant.
When such a cosmetic composition is applied to skin, hair or nails, the heat of vaporization is deprived due to volatilization of the solvent A in a coating film, and the moisture in air is condensed on the surface of the coating film and attached thereto as fine waterdrops. In the present invention, the solvent A is at least one selected from specific alcohols, and the distance Ra of the solubility parameter of the solvent B to water is a specific value or more, and therefore, the solvent B having been compatibilized with the solvent A owing to adhesion of the fine waterdrops to the surface of the coating film causes phase separation. Then, it may be considered that since the polymer C is insoluble in the solvent B, the polymer C coats the phase-separated solvent B, and coalescence of the solvent B is suppressed, whereby primary particles containing a colorant and having a core-shell structure in which the solvent B constitutes the core and the polymer C constitutes the shell are formed. Furthermore, it may be considered that following the volatilization of the solvent A and the surface alignment of the formed primary particles, a cell-shaped convection structure regularly divided within the coating film, so-called "Benard cells", is generated, the primary particles are accumulated due to the Benard convection in each cell to form secondary particles, whereby the cosmetic coating film containing a colorant is obtained. As a result, it may be conjectured that the light is scattered due to the particle structure formed in the cosmetic coating film, and high concealing performance and coloring performance can be thereby expressed.

<Solvent A>

The cosmetic composition of the present invention contains the solvent A.

The solvent A is at least one selected from the group consisting of ethanol, n-propanol and isopropanol. Further, in the cosmetic composition, the solvent A is compatible with the solvent B and dissolves the polymer C. With that, on the occasion of applying the cosmetic composition to skin, hair or nails, the heat of vaporization is deprived following volatilization of the solvent A, fine waterdrops attach onto the surface of the coating film, thereby enabling one to cause phase separation between the solvent A and the solvent B.

The solvent A may be used alone or in combination of two or more thereof.

The solvent A is, from the viewpoint of improving concealing performance and coloring performance, preferably at least one selected from the group consisting of ethanol and isopropanol, more preferably ethanol.

<Solvent B>

The cosmetic composition of the present invention contains the solvent B.

As for the solvent B, its boiling point is 150°C or higher, and the distance Ra of the Hansen solubility parameter of the solvent B to water as expressed by the aforementioned equation (1) is 40 or more. Furthermore, in the cosmetic composition, the solvent B is compatible with the solvent A and does not dissolve the polymer C therein. According to this, on the occasion when fine waterdrops attach onto the surface of the coating film due to volatilization of the solvent A, phase separation between the solvent A and the solvent B is caused, and the primary particles in which the solvent B is coated with the polymer C are formed.

The boiling point of the solvent B is 150°C or higher, preferably 155°C or higher, more preferably 160°C or higher, still more preferably 165°C or higher, and yet still more preferably 170°C or higher from the viewpoint of forming primary particles and improving concealing performance and coloring performance, and is preferably 300°C or lower, more preferably 270°C or lower, still more preferably 250°C or lower, yet still more preferably 230°C or lower, even yet still more preferably 210°C or lower, and even still more preferably 180°C or lower from the viewpoint of handling properties.

The distance Ra of the Hansen solubility parameter of the solvent B to water is 40 or more, preferably 42 or more, and more preferably 44 or more, and is preferably 60 or less, more preferably 57 or less, still more preferably 55 or less, yet still more preferably 50 or less, from the viewpoint of forming primary particles and improving concealing performance and coloring performance.

The solvent B may be used alone or in combination of two or more thereof.

In the case of using the solvent B in combination of two or more thereof, the boiling point of the solvent B and the distance Ra of the Hansen solubility parameter of the solvent B to water can be determined as a weighted average value resulting through weighing in terms of the content (% by mass) of each of the solvents.

From the viewpoint of improving concealing performance and coloring performance, the solvent B preferably contains at least one selected from the group consisting of a hydrocarbon oil and a silicone oil.

Examples of the hydrocarbon oil include $\alpha$-olefin oligomers; liquid paraffins; liquid isoparaffins (light liquid isoparaffins, heavy liquid isoparaffins), such as isododecane, isohexadecane and hydrogenated polyisobutene; liquid ozokerite; squalane; pristane; and squalene. Above all, the hydrocarbon oil is preferably at least one selected from the group consisting of $\alpha$-olefin oligomers, liquid paraffins, liquid isoparaffins, liquid ozokerite, squalane, pristane, and squalene, more preferably liquid isopararffins, still more preferably at least one selected from the group consisting of isododecane, isohexadecane and hydrogenated polyisobutene, yet still more preferably at least one selected from the group consisting of isododecane and hydrogenated polyisobutene.

A weight average molecular weight of the hydrocarbon oil is preferably 150 or more, and more preferably 160 or more, and is preferably 1,000 or less, more preferably 500 or less, and still more preferably 300 or less.

A viscosity at 20°C of the hydrogenated polyisobutene is preferably 0.5 mPa·s or more, more preferably 0.7 mPa·s or more, and still more preferably 1 mPa·s or more, and is preferably 30 mPa·s or less, more preferably 25 mPa·s or less, still more preferably 20 mPa·s or less, yet still more preferably 15 mPa·s or less, even yet still more preferably 10 mPa·s or less, even still more preferably 5 mPa·s or less, further more preferably 3 mPa·s or less, further more preferably 2 mPa·s or less. The viscosity at 20°C of the hydrogenated polyisobutene can be measured with an E-type viscometer by the method described in the section of Examples.

Examples of the silicone oil include linear silicone oils, such as trisiloxane; branched silicone oils, such as methyltrimethicone; and cyclic silicone oils, such as methylcyclopolysiloxane. Above all, the silicone oil is preferably at least one selected from the group consisting of linear silicone oils, branched silicone oils and cyclic silicone oils, more preferably at least one selected from the group consisting of trisiloxane, methyltrimethicone and methylcyclopolysiloxane, still more preferably at least one selected from the group consisting of trisiloxane and methyltrimethicone.

A weight average molecular weight of the silicone oil is preferably 150 or more, and more preferably 160 or

more, and is preferably 1,000 or less, more preferably 500 or less, and still more preferably 300 or less.

A viscosity at 25°C of the silicone oil is preferably 0.5 mPa·s or more, and is also preferably 20 mPa·s or less, more preferably 10 mPa·s or less, still more preferably 5 mPa·s or less, yet still more preferably 3 mPa·s or less, even yet still more preferably 2 mPa·s or less. The viscosity at 25°C of the silicone oil can be measured with an E-type viscometer by the method described in the section of Examples.

The solvent B is, from the viewpoint of improving formability of cosmetic coating films and improving concealing performance and coloring performance thereof, preferably volatile, and is more preferably at least one selected from the group consisting of volatile hydrocarbon oils and volatile silicone oils.

In the present invention, the wording "volatile" means that the evaporation amount at 25°C for 6 hours, as measured according to the following method, is 20% or more.

Measurement method: A filter paper disc having a diameter of 90 mm is put in a glass-made laboratory dish having a diameter of 120 mm, 1 g of a sample is put on the filter paper disc and stored in room (25°C) at 65% RH for 6 hours. The mass of the sample before and after storage is measured, and the evaporation amount is calculated according to the following equation.

$$\text{Evaporation amount (\%)} = [(\text{mass of sample before storage} - \text{mass of sample after storage})/\text{mass of sample before storage}] \times 100$$

In the present invention, as described above, primary particles having a core-shell structure where the solvent B is the core and the polymer C is the shell are formed, and since the solvent B is volatile, the solvent B encapsulated in the core part is evaporated away to form hollow primary particles, and accordingly, concealing performance and coloring performance, as well as film formability of cosmetic coating films can be improved.

In forming the hollow primary particles, fine pores (openings) may be formed in the shell part. In such a case, for example, in application of the cosmetic composition of the present invention to skin, even though skin depressions or wrinkles are deformed by change in facial expression, the hollow primary particles taken inside the skin depressions or wrinkles can be reversibly restored to the original state with following the volume change of the skin depressions or wrinkles by deformation of the particles or by pushing out the internal air. Consequently, it is considered that pushing out of the hollow primary particles onto the skin surface can be suppressed, the condition of the cosmetic coating film before deformation of skin depressions and wrinkles can be kept good, and the makeup retention can be thereby improved. Preferably, the hollow primary particles are formed by controlling the kind of the solvent B and controlling the drying condition of the coating film formed on skin to evaporate the solvent B encapsulated in the core part.

The volatile hydrocarbon oil is, from the viewpoint of improving concealing performance and coloring performance and formability of cosmetic coating films, preferably a saturated or unsaturated hydrocarbon oil having 8 or more and 16 or less carbon atoms. Examples of the volatile hydrocarbon oil include paraffin-type hydrocarbon oils such as n-decane, n-undecane and n-dodecane; isoparaffin-type hydrocarbon oils such as isodecane, isododecane, and hydrogenated polyisobutene (light liquid isoparaffin); and cyclic paraffin hydrocarbon oils such as cyclodecane and cyclododecane. Among these, the volatile hydrocarbon oil is preferably a liquid isoparaffin, more preferably at least one selected from the group consisting of isodecane, isododecane and hydrogenated polyisobutene, still more preferably at least one selected from the group consisting of isododecane and hydrogenated polyisobutene, yet still more preferably hydrogenated polyisobutene.

Commercial products of the volatile hydrocarbon oil include "PARLEAM 3" and "PARLEAM 4" by NOF Corporation; and Marukasol R by Maruzen Petrochemical Co., Ltd.

The volatile silicone oil is, from the viewpoint of improving concealing performance and coloring performance as well as formability of cosmetic coating films, preferably at least one selected from the group consisting of a linear organopolysiloxane and cyclic organopolysiloxane.

Specific examples of the linear organopolysiloxane include octamethyltrisiloxane, decamethyltetrasiloxane, dodecamethylpentasiloxane, and 1,1,1,3,5,5,5-heptamethyl-3-[(trimethylsilyl)oxy]-trisiloxane.

Examples of cyclic organopolysiloxane include a 4- to 6-membered cyclic siloxane having a substituent of an alkyl group having 1 or more and 5 or less carbon atoms. Specific examples of the cyclic organopolysiloxane include octamethylcyclotetrasiloxane, decamethylcyclopentasiloxane, and dodecamethylcyclohexasiloxane.

Commercial products of the volatile silicone oil include "KF-96A-1cs" (octamethyltrisiloxane), "KF-96L-1.5cs" (decamethyltetrasiloxane), "KF-96L-2cs" (dodecamethylpentasiloxane), "KF-995" (decamethylcyclopentasiloxane) and "TMF-1.5" (1,1,1,3,5,5,5-heptamethyl-3-[(trimethylsilyl)oxy]-trisiloxane) by Shin-Etsu Chemical Co., Ltd.; "SH200C Fluid 1cs" (octamethyltrisiloxane), "SH200C Fluid 1.5cs" (decamethyltetrasiloxane), "SH200C Fluid 2cs" (dodecamethylpentasiloxane) and "SH245 Fluid"(decamethylcyclopentasiloxane) by Dow Corning Toray Co., Ltd.; and "TSF405A" (decamethylcyclopentasiloxane) by Momentive Performance Materials Corporation.

The solvent B may also be one containing, in addition to the hydrocarbon oil or the silicone oil, an additive, such as a moisturizer, an ultraviolet absorber, a pest repellent, a wrinkling-preventing agent, and a fragrance.

In the case where the solvent B contains at least one selected from the group consisting of a hydrocarbon oil and a silicone oil each having a weight average molecular weight of 150 or more and 1,000 or less, the content of at least one selected from the group consisting of a hydrocarbon oil and a silicone oil each having a weight average molecular weight of 150 or more and 1,000 or less in the solvent B is, from the viewpoint of improving concealing performance and coloring performance, preferably 50% by mass or more, more preferably 70% by mass or more, still more preferably 90% by mass or more, and is preferably 100% by mass or less, more preferably 100% by mass.

<Polymer C>

In the present invention, the polymer C coats the phase-separated solvent B and contributes to the formation of primary particles.

The polymer C is not particularly restricted so long as it is soluble in the solvent Abut insoluble in the solvent B.

Examples of the polymer C include ionic polymers, such as an anionic polymer, a cationic polymer, and a betaine polymer; and nonionic polymers.

(Anionic Polymer)

The anionic polymer has an anionic group. Examples of the anionic group include groups that are capable of releasing a hydrogen ion upon dissociation thereof to exhibit acidity, such as a carboxy group (-COOM), a sulfonic acid group ($-SO_3M$), and a phosphoric acid group (-OPOsMs), or dissociated ion forms of these groups (such as $-COO^-$, $-SO_3^-$, $-OPO_3^{2-}$, and $-OPO_3^-M$). In the aforementioned chemical formulae, M represents a hydrogen atom, an alkali metal, ammonium, or an organic ammonium.

The anionic polymer is, from the viewpoint of improving concealing performance and coloring performance, preferably an anionic polymer CI containing a structural unit derived from a monomer having an acid group (hereinafter also referred to as "anionic polymer CI").

The monomer having an acid group is, from the same viewpoint as above, preferably a monomer having a carboxy group, more preferably at least one selected from the group consisting of (meth)acrylic acid, crotonic acid, itaconic acid, maleic acid, fumaric acid, citraconic acid, and 2-methacryloyloxymethyl succinic acid, and still more preferably (meth)acrylic acid.

Here, the term "(meth)acrylic acid" means at least one selected from the group consisting of acrylic acid and methacrylic acid.

The anionic polymer CI is, from the viewpoint of improving concealing performance and coloring performance, preferably a copolymer further containing a structural unit derived from other monomer than the monomer having an acid group. Examples of the other monomer include hydrophobic monomers, such as a (meth)acrylate having a hydrocarbon group derived from an aliphatic alcohol and an aromatic group-containing monomer; and nonionic monomers.

Here, the term "(meth)acrylate" means at least one selected from the group consisting of acrylates and methacrylates.

The (meth)acrylate having a hydrocarbon group derived from an aliphatic alcohol is one having a hydrocarbon group derived from an aliphatic alcohol having preferably 1 or more and 22 or less carbon atoms, more preferably 1 or more and 12 or less carbon atoms, and still more preferably 1 or more and 8 or less carbon atoms. Examples of the (meth)acrylate include a (meth)acrylate having a linear alkyl group; a (meth)acrylate having a branched alkyl group; and a (meth)acrylate having an alicyclic alkyl group.

The aromatic group-containing monomer is preferably a vinyl monomer having an aromatic group having 6 or more and 22 or less carbon atoms, which may have a substituent containing a hetero atom, and more preferably at least one selected from the group consisting of a styrene-based monomer and an aromatic group-containing (meth)acrylate. A molecular weight of the aromatic group-containing monomer is preferably less than 500.

Examples of the styrene-based monomer include styrene, $\alpha$-methylstyrene, 2-methylstyrene, vinyltoluene, and divinylbenzene.

Examples of the aromatic group-containing (meth)acrylate include phenyl (meth)acrylate, benzyl (meth)acrylate, and phenoxyethyl (meth)acrylate.

Examples of the nonionic monomer in the anionic polymer CI include (meth)acrylamide; N-vinyl-2-pyrrolidone; linear, branched or cyclic alkyl group-having N-alkyl(meth)acrylamides such as N-tert-butylacrylamide, N-tert-octylacrylamide, N-(2-ethylhexyl)acrylamide, N-n-octylacrylamide, N-dodecylacrylamide, N-n-heptylacrylamide, N-hexylacrylamide, and N-cyclohexylmethacrylamide; hydroxyalkyl (meth)acrylates; polyalkylene glycol (meth)acrylates (n = 2 to 30, n represents an average addition molar number of the oxyalkylene group; hereinafter the same); alkoxypolyalkylene glycol (meth)acrylates (n = 1 to 30); phenoxypolyalkylene glycol (meth)acrylates such as phenoxy(ethylene glycol/propylene glycol copolymer) (n = 1 to 30, in which n for ethylene glycol: n = 1 to 29) (meth)acrylates.

Specific examples of commercially available nonionic monomers include NK ESTER M-20G, NK ESTER M-40G, NK ESTER M-90G, NK ESTER M-230G, all of which are manufactured by Shin-Nakamura Chemical Co., Ltd.; and BLEMMER PE-90, BLEMMER PE-200, BLEMMER PE-350, BLEMMER PME-100, BLEMMER PME-200, BLEMMER PME-400, BLEMMER PP-500, BLEMMER PP-800, BLEMMER PP-1000, BLEMMER AP-150, BLEMMER AP-400, BLEMMER AP-550, BLEMMER 50PEP-300, BLEMMER 50POEP-800B, BLEMMER 43PAPE-600B, all of which are manufactured by NOF Corporation.

Each of the aforementioned monomers can be used alone or in combination of two or more thereof.

A weight average molecular weight of the anionic polymer CI is preferably 5,000 or more, more preferably 10,000 or more, and still more preferably 20,000 or more, and is preferably 1,000,000 or less, more preferably 500,000 or less, and still more preferably 200,000 or less. The weight average molecular weight of the anionic polymer CI is a molecular weight measured by the gel permeation chromatography (GPC) as expressed in terms of polystyrene.

Examples of commercially available products of the anionic polymer CI include acrylic acid/acrylic acid alkyl ester/(N-alkyl) acrylamide copolymers, such as ULTRAHOLD 8, ULTRAHOLD STRONG, and ULTRAHOLD POWER by BASF Japan Ltd., and AMPHOMER V-42 by National Starch & Chemical Co.; carboxyvinyl polymers, such as CARBOPOL series by Lubrizol Advanced Materials, Inc.; (meth)acrylic acid/(meth)acrylic acid alkyl ester copolymers, such as DIAHOLD by Mitsubishi Chemical Corporation; ((meth)acrylic acid/diacetone acrylamide) copolymer AMP, ((meth)acrylic acid/acrylic acid alkyl ester/diacetone acrylamide) copolymer AMP, and ((meth)acrylic acid/(meth)acrylic acid alkyl ester/(N-alkyl)alkylacrylamide) copolymer AMP, such as PLASCIZE series by Goo Chemical Co., Ltd.; and (meth)acrylic acid/acrylic acid alkyl ester/vinylpyrrolidone copolymers, such as LUVIFLEX VBM35 by BASF SE.

In addition, also usable for cosmetics are commercial products of a polymer having a structural unit derived from acrylic acid or methacrylic acid as an acid group-having monomer, such as Aniset series by Osaka Organic Chemical Industry Ltd.

Here, "(meth)acrylic acid alkyl ester" means at least one selected from the group consisting of an acrylic acid alkyl ester and a methacrylic acid alkyl ester.

The anionic polymer CI is, from the viewpoint of improving concealing performance and coloring performance, preferably a copolymer containing a structural unit derived from a monomer having an acid group and a structural unit derived from a (meth)acrylic acid alkyl ester; more preferably a copolymer containing a structural unit derived from a monomer having an acid group, a structural unit derived from a (meth)acrylic acid alkyl ester, and a structural unit derived from an (N-alkyl) (meth)acrylamide; still more preferably a (meth)acrylic acid/(meth)acrylic acid alkyl ester/(N-alkyl) (meth)acrylamide copolymer; and yet still more preferably an acrylic acid/acrylic acid alkyl ester/(N-alkyl) acrylamide copolymer.

(Cationic Polymer)

In the present invention, the wording "cationic" of the cationic polymer means that in the case where a non-neutralized polymer is dispersed or dissolved in pure water, the pH becomes larger than 7; in the case of a polymer having a quaternary ammonium group or the like, when it is dispersed or dissolved in pure water while making its counter ion as a hydroxide ion, the pH becomes larger than 7; or in the case where a polymer is insoluble in pure water, and the pH cannot be distinctly measured, a zeta potential of the dispersion of the polymer dispersed in pure water becomes positive.

The cationic polymer preferably has, from the viewpoint of improving concealing performance and coloring performance, a basic group, such as a primary, secondary, or tertiary amino group, a quaternary ammonium group, and a hydrazino group, and more preferably has a quaternary ammonium group.

The basic group includes ones neutralized with an acid, such as hydrochloric acid, sulfuric acid, nitric acid, acetic acid, formic acid, maleic acid, fumaric acid, citric acid, tartaric acid, adipic acid, and lactic acid.

Examples of the cationic polymer include a natural cationic polymer and a synthetic cationic polymer.

The natural cationic polymer is a polymer obtained through an operation, such as extraction and purification, from a natural product or one resulting through chemical modification of the foregoing polymer, and examples thereof include ones having a glucose residue in the polymer skeleton. Specifically, examples thereof include cationized guar gum, cationized tara gum, cationized locust bean gum, cationized cellulose, a cationized hydroxyalkyl cellulose, and a cationic starch.

Examples of the synthetic cationic polymer include polyethyleneimine, polyallylamine or an acid-neutralized product thereof, a polyglycol-polyamine condensate, cationic polyvinyl alcohol, cationic polyvinylpyrrolidone, a cationic silicone polymer, a 2-(dimethylamino)ethyl methacrylate polymer or an acid-neutralized product thereof, poly(trimethyl-2-methacryloyloxyethylammonium chloride), an amine/epichlorohydrin copolymer, an N,N-dimethylaminoethyl methacrylic acid diethyl sulfate/vinylpyrrolidone copolymer, an N,N-dimethylaminoethyl methacrylic acid diethyl sulfate/N,N-dimethyl acrylamide/dimethacrylic acid polyethylene glycol copolymer, polydiallyldimethylammonium chloride, a diallyldimethylammonium chloride/acrylamide copolymer, a diallyldimethylammonium chloride/sulfur dioxide copolymer, a diallyldimethylammonium chloride/hydroxyethyl cellulose copolymer, a 1-allyl-3-methylimidazolium chloride/vinylpyrrolidone copolymer, an alkylamino (meth)acrylate/vinylpyrrolidone copolymer, an alkylamino (meth)acrylate/vinylpyrrolidone/vinyl caprolactam copolymer, a (3-(meth)acrylamidopropyl)trimethylammonium chloride/vinylpyrrolidone copolymer, and an alkylaminoalkyl acrylamide/alkyl acrylamide/(meth)acrylate/polyethylene glycol (meth)acrylate copolymer.

These cationic polymers can be used alone or in combination of two or more thereof.

Among these, the cationic polymer is, from the viewpoint of improving concealing performance and coloring performance, preferably a cationic polymer CII-1 containing a structural unit derived from a monomer having a basic group (hereinafter also referred to as "cationic polymer CII-1") and a cationic silicone polymer (hereinafter also referred to as "cationic silicone polymer CII-2").

[Cationic Polymer CII-11

The cationic polymer CII-1 contains a structural unit derived from a monomer having a basic group. Examples of the foregoing basic group include the same groups as mentioned above.

Examples of the monomer having a basic group include amino group-containing monomers, such as an alkylamino (meth)acrylate, an N,N-dialkylaminoalkyl (meth)acrylate, N-[3-(dimethylamino)propyl] (meth)acrylamide, and a diallyldialkylammonium, and acid-neutralized products or quaternized products thereof. These basic group-having monomers can be used alone or in combination of two or more thereof.

Examples of the acid for acid neutralization include hydrochloric acid, sulfuric acid, nitric acid, acetic acid, formic acid, maleic acid, fumaric acid, citric acid, tartaric acid, adipic acid, and lactic acid, and examples of the quaternizing agent include alkyl halides, such as methyl chloride, ethyl chloride, methyl bromide, and methyl iodide; and alkylating agents, such as dialkyl sulfates, e.g., dimethyl sulfate, diethyl sulfate, and di-n-propyl sulfate.

The cationic polymer CII-1 is, from the viewpoint of improving concealing performance and coloring performance, preferably a homopolymer of a monomer having a basic group, a copolymer of a monomer having a basic group and other monomer than the foregoing monomer having a basic group, or a polycondensate; more preferably a copolymer of a monomer having a basic group and other monomer than the foregoing monomer having a basic group; still more preferably a copolymer containing a structural unit derived from a monomer having a basic group, a structural unit derived from the hydrophobic group as exemplified above for the anionic polymer CI, and a structural unit derived from the nonionic monomer as exemplified above for the anionic polymer CI; yet still more preferably a copolymer containing a structural unit derived from an amino group-containing monomer, a structural unit derived from a (meth)acrylic acid alkyl ester, a structural unit derived from an N-alkyl (meth)acrylamide, and a structural unit derived from an alkoxy polyethylene glycol mono(meth)acrylate; even yet still more preferably a copolymer containing a structural unit derived from an amino group-containing monomer, a structural unit derived from a (meth)acrylic acid alkyl ester having a hydrocarbon group derived from an aliphatic alcohol having 1 or more and 22 or less carbon atoms, a structural unit derive from an N-alkyl(meth)acrylamide having a linear, branched or cyclic alkyl group, and a structural unit derived from an alkoxypolyethylene glycol mono(meth)acrylate; and even still more preferably a copolymer containing a structural unit derived from an amino group-containing monomer, a structural unit derived from a (meth)acrylic acid alkyl ester having a hydrocarbon group derived from an aliphatic alcohol having 1 or more and 8 or less carbon atoms, a structural unit derive from an N-alkyl(meth)acrylamide having a branched alkyl group having 4 or more and 8 or less carbon atoms, and a structural unit derived from a methoxypolyethylene glycol mono(meth)acrylate.

The cationic polymer CII-1 is produced by copolymerizing raw material monomers containing these monomers by a known polymerization method, such as a bulk polymerization method, a solution polymerization method, a suspension polymerization method, and an emulsion polymerization method. Of these polymerization methods, a solution polymerization method is preferred.

From the viewpoint of improving concealing performance and coloring performance, at the time of producing the cationic polymer CII-1, the content of the monomer having a basic group, the hydrophobic monomer, and the nonionic monomer in the raw material monomers (the content as the non-neutralized content, hereafter the same), namely, the content of the structural unit derived from each of the components in the cationic polymer CII-1 is as follows.

The content of the monomer having a basic group is preferably 3% by mass or more, more preferably 5% by mass or more, and still more preferably 7% by mass or more, and is preferably 35% by mass or less, more preferably 30% by mass or less, still more preferably 25% by mass or less, yet still more preferably 20% by mass or less, and even yet still more preferably 17% by mass or less.

The content of the hydrophobic monomer is preferably 5% by mass or more, more preferably 10% by mass or more, and still more preferably 15% by mass or more, and is preferably 35% by mass or less, more preferably 30% by mass or less, and still more preferably 25% by mass or less.

The content of the nonionic monomer is preferably 30% by mass or more, more preferably 40% by mass or more, still more preferably 50% by mass or more, and yet still more preferably 55% by mass or more, and is preferably

85% by mass or less, more preferably 80% by mass or less, and still more preferably 75% by mass or less.

A weight average molecular weight of the cationic polymer CII-1 is preferably 7,000 or more, more preferably 10,000 or more, still more preferably 50,000 or more, and yet still more preferably 100,000 or more, and is preferably 500,000 or less, more preferably 300,000 or less, still more preferably 200,000 or less, and yet still more preferably 150,000 or less, from the viewpoint of improving concealing performance and coloring performance.

The weight average molecular weight of the cationic polymer CII-1 can be measured by the method described in the section of Examples.

[Cationic Silicone Polymer CII-2]

The cationic silicone polymer CII-2 is preferably a poly(N-acylalkyleneimine)/organopolysiloxane copolymer containing an organopolysiloxane segment (x) (hereinafter also referred to simply as "segment (x)") and a poly(N-acylalkyleneimine) segment (y) composed of an alkylene group containing a cationic nitrogen atom binding to at least one silicon atom of the segment (x) and a repeating unit of an N-acylalkyleneimine represented by following general formula (1-1) (the poly(N-acylalkyleneimine) segment (y) will be hereinafter also referred to simply as "segment (y)").

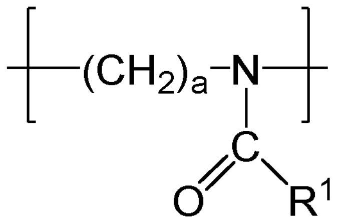

(1-1)

In the formula, $R^1$ represents a hydrogen atom, an alkyl group having 1 or more and 22 or less carbon atoms, an aryl group having 6 or more and 22 or less carbon atoms, or an arylalkyl group or alkylaryl group having 7 or more and 22 or less carbon atoms; and a is 2 or 3.

In the general formula (1-1), the alkyl group represented by $R^1$ is preferably an alkyl group having 1 or more and 12 or less carbon atoms, more preferably an alkyl group having 1 or more and 3 or less carbon atoms, such as a methyl group, an ethyl group, an n-propyl group, and an isopropyl group and still more preferably an ethyl group.

Examples of the aryl group represented by $R^1$ include a phenyl group and a naphthyl group.

Examples of the arylalkyl group represented by $R^1$ include a phenylalkyl group and a naphthylalkyl group, in which the carbon number of the alkyl group is 1 or more and 20 or less; and examples of the alkylaryl group include an alkylphenyl group and an alkylnaphthyl group, in which the carbon number of the alkyl group is 1 or more and 20 or less.

In the general formula (1-1), a is preferably 2.

Although a degree of polymerization of the repeating unit represented by the general formula (1-1) in the segment (y) is not particularly restricted, for example, it is preferably 1 or more and 500 or less, and more preferably 6 or more and 100 or less.

Examples of the organopolysiloxane that forms the segment (x) include compounds represented by the following general formula (1-2).

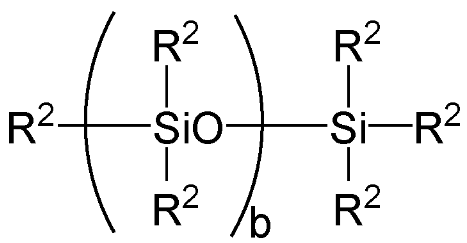

(1-2)

In the formula, $R^2$ represents an alkyl group having 1 or more and 22 or less carbon atoms, a phenyl group, or an alkyl group containing a nitrogen atom; though a plurality of the $R^2$ may be the same as or different from each other, at least one of them is an alkyl group containing a cationic nitrogen atom; and b is 100 or more and 5,000 or less.

In the general formula (1-2), among the alkyl groups having 1 or more and 22 or less carbon atoms as represented by $R^2$, an alkyl group having 1 or more and 12 or less carbon atoms is preferred, an alkyl group having 1 or more and 3 or less carbon atoms is more preferred, and a methyl group is still more preferred.

Examples of the alkyl group containing a nitrogen atom as represented by $R^2$ include alkyl groups having 2 or

more and 20 or less carbon atoms which contains preferably 1 or more and 3 or less nitrogen atoms. The alkyl group containing a nitrogen atom may be existent in at least one silicon atom at an end or in a side chain of the organopolysiloxane, and the number of alkyl groups containing a nitrogen atom in the organopolysiloxane is preferably 1 or more and 300 or less, and more preferably 1 or more and 100 or less.

In the general formula (1-2), b is preferably 100 or more and 2,000 or less, and more preferably 350 or more and 1,500 or less.

A weight average molecular weight of the organopolysiloxane that forms the segment (x) is preferably 1,000 or more, more preferably 10,000 or more, and still more preferably 30,000 or more, and is preferably 1,000,000 or less, more preferably 500,000 or less, still more preferably 200,000 or less, and yet still more preferably 150,000 or less.

Examples of the alkylene group containing a nitrogen atom intervening in the bonding between the segment (x) and the segment (y) include alkylene groups having 2 or more and 20 or less carbon atoms which contains preferably 1 or more and 3 or less nitrogen atoms.

Specifically, examples of the nitrogen atom existing between carbon and carbon of the alkylene chain or at an end of the alkylene chain include (i) a secondary amine or a tertiary amine, (ii) an ammonium salt in which a hydrogen ion is added to a secondary amine or a tertiary amine, and (iii) a quaternary amine salt.

The poly(N-acylalkyleneimine)/organopolysiloxane copolymer is preferably one in which the segment (y) is bound to at least one silicon atom at an end or in a side chain of the segment (x) via the alkylene group containing a cationic nitrogen atom.

A mass ratio of the content of the segment (x) to the total content of the segment (x) and the segment (y) [{content of segment (x)}/{total content of segment (x) and segment (y)}] in the poly(N-acylalkyleneimine)/organopolysiloxane copolymer is preferably 0.1 or more, more preferably 0.3 or more, still more preferably 0.4 or more, and yet still more preferably 0.5 or more, and is preferably 0.99 or less, more preferably 0.95 or less, and still more preferably 0.9 or less, from the viewpoint of improving concealing performance and coloring performance.

In this specification, the mass ratio [{content of segment (x)}/{total content of segment (x) and segment (y)}] is a ratio of a mass (Mx) of the segment (x) to the total amount of a mass (Mx) of the segment (x) and a mass (My) of the segment (y) in the poly(N-acylalkyleneimine)/organopolysiloxane copolymer.

The mass ratio [{content of segment (x)}/{total content of segment (x) and segment (y)}] can be determined by dissolving 5% by mass of the poly(N-acylalkyleneimine)/organopolysiloxane copolymer in deuterated chloroform and calculating an integration ratio of an alkyl group or a phenyl group in the segment (x) and a methylene group in the segment (y) through a nuclear magnetic resonance ($^1$H-NMR) analysis.

A weight average molecular weight of the poly(N-acylalkyleneimine)/organopolysiloxane copolymer is preferably 10,000 or more, more preferably 50,000 or more, and still more preferably 70,000 or more, and is preferably 1,000,000 or less, more preferably 500,000 or less, and still more preferably 200,000 or less, from the viewpoint of improving concealing performance and coloring performance. The weight average molecular weight of the poly(N-acylalkyleneimine)/organopolysiloxane copolymer can be calculated from the weight average molecular weight of the organopolysiloxane that forms the segment (x) and the aforementioned mass ratio [{content of segment (x)}/{total content of segment (x) and segment (y)}].

Suitable examples of the poly(N-acylalkyleneimine)/organopolysiloxane copolymer include a poly(N-formylethyleneimine)/organopolysiloxane copolymer, a poly(N-acetylethyleneimine)/organopolysiloxane copolymer, and a poly(N-propionylethyleneimine)/organopolysiloxane copolymer.

The poly(N-acylalkyleneimine)/organopolysiloxane copolymer can be, for example, obtained by a method of allowing the (N-acylalkyleneimine) that is a ring-opening polymer of a cyclic imino ether and the organopolysiloxane that forms the segment (x) to react with each other. More specifically, the poly(N-acylalkyleneimine)/organopolysiloxane copolymer can be, for example, obtained by the method described in JP 2011-126978 A. The poly(N-acylalkyleneimine)/organopolysiloxane copolymer to be used as the cationic silicone polymer CII-2 can be used alone or in combination of two or more thereof.

[Betaine Polymer]

In the present invention, examples of the betaine polymer include a copolymer of a monomer having an anionic group and a monomer having a cationic group, a polymer or copolymer of a betaine monomer, a polymer having an anionic group introduced into a cationic polymer, and a polymer having the aforementioned basic group introduced into an anionic polymer. Above all, from the viewpoint of improving concealing performance and coloring performance, the betaine polymer is preferably a polymer containing a betaine structure in a side chain thereof, and is more preferably a betaine polymer containing a structural unit derived from a betaine monomer (hereinafter also referred to as "betaine polymer CIII).

The betaine monomer is, from the viewpoint of improving concealing performance and coloring performance, preferably a monomer containing a betaine structure and a (meth)acrylamide structure, more preferably at least one

selected from the group consisting of a carboxybetaine monomer, a sulfobetaine monomer, and a phosphobetaine monomer, and still more preferably a carboxybetaine monomer.

Examples of the betaine polymer include polymethacryloylethyl dimethylbetaine, (a homopolymer of N-methacryloyloxyethyl-N,N-dimethylammonium-α-N-methylcarboxybetaine), an N-methacryloyloxyethyl-N,N-dimethylammonium-α-N-methylcarboxybetaine/methacrylic acid alkyl ester copolymer, a methacryloylethyldimethylbetaine/methacryloylethyltrimethylammonium chloride/2-hydroxyethyl methacrylate copolymer, a methacryloylethyldimethylbetaine/methacryloyethyltrimethylammonium chloride/methoxy polyethylene glycol methacrylate copolymer, and an octylacrylamide/(meth)acrylic acid or (meth)acrylic acid alkyl ester/butylaminoethyl methacrylate copolymer. Above all, the betaine polymer CIII is, from the viewpoint of improving concealing performance and coloring performance, preferably a copolymer containing a structural unit derived from a betaine monomer and a structural unit derived from a (meth)acrylic acid alkyl ester having a hydrocarbon group derived from an aliphatic alcohol having 1 or more and 22 or less carbon atoms, more preferably a copolymer containing a structural unit derived from a carboxybetaine monomer and a structural unit derived from a (meth)acrylic acid alkyl ester having a hydrocarbon group derived from an aliphatic alcohol having 1 or more and 22 or less carbon atoms, and still more preferably an N-methacryloyloxyethyl-N,N-dimethylammonium-α-N-methylcarboxybetaine/methacrylic acid alkyl ester copolymer.

A weight average molecular weight of the betaine polymer is preferably 5,000 or more, and more preferably 10,000 or more, and is preferably 1,000,000 or less, more preferably 500,000 or less, and still more preferably 300,000 or less, from the viewpoint of improving concealing performance and coloring performance. The weight average molecular weight of the betaine polymer is a molecular weight measured by the gel permeation chromatography (GPC) as expressed in terms of polystyrene.

Examples of commercially available betaine polymers include PLASCIZE L-410W, PLASCIZE L-402W, PLASCIZE L-440, PLASCIZE L-440W, PLASCIZE K-450, and PLASCIZE L-450W (all of which are a trade name, manufactured by Goo Chemical Co., Ltd.); YUKA FORMER SM and YUKA FORMER 301 (all of which are a trade name, manufactured by Mitsubishi Chemical Corporation); RAM RESIN-1000, RAM RESIN-2000, RAM RESIN-3000, and RAM RESIN-4000 (all of which are a trade name, manufactured by Osaka Organic Chemical Industry Ltd.); MERQUAT PLUS 3330 (a trade name, manufactured by Lubrizol Japan Ltd.); and UNFOAMER 28-4910 and UNFOAMER LV-71 (all of which are a trade name, manufactured by Akzo Nobel N.V).

[Nonionic Polymer]

Examples of the nonionic polymer include polymers having a structural unit derived from a nonionic monomer; and water-soluble polysaccharides (such as a cellulose-based polymer, a gum-based polymer, and a starch-based polymer) and derivatives thereof.

Examples of the nonionic monomer in the nonionic polymer include (meth)acrylates having a hydrocarbon group derived from an aliphatic alcohol having 1 or more and 22 or less carbon atoms; N-vinyl-2-pyrrolidone; vinyl alcohol; polyalkylene glycol (meth)acrylates (n = 1 to 30); alkoxypolyalkylene glycol mono(meth)acrylates (n = 1 to 30); and (meth)acrylamides and derivatives thereof.

The nonionic polymer may further contain a structural unit derived from other monomer than the nonionic monomer. Examples of the other monomer include the aforementioned styrene-based monomers; the aforementioned aromatic group-containing (meth)acrylates; and vinyl acetate.

Specifically, examples of the nonionic polymer include polyvinyl alcohol, polyvinyl acetal, polyurethane-polyurea, polyvinylpyrrolidone, copolymers of vinylpyrrolidone and other nonionic monomer, such as a vinylpyrrolidone/vinyl acetate copolymer, cellulose-based polymers, such as a hydroxyalkyl cellulose, polyethylene glycol, polypropylene glycol, polyglycerin, polyvinyl alcohol, pullulan, guar gum, poly-N,N-dimethylacrylamide, poly-N-vinylacetamide, poly-N-vinylformamide, and poly(2-alkyl-2-oxazoline). Above all, the nonionic polymer is, from the viewpoint of improving concealing performance and coloring performance, preferably polyvinyl acetal and polyurethane-polyurea are preferred.

In the case where polyvinyl butyral is used as polyvinyl acetal, the acetalization degree of polyvinyl butyral is, from the viewpoint of improving concealing performance and coloring performance, preferably 50 mol% or more, more preferably 55 mol% or more, still more preferably 60 mol% or more, and is preferably 80 mol% or less, more preferably 75 mol% or less, still more preferably 70 mol% or less.

Examples of commercially available nonionic polymers include polyvinyl butyral, such as S-LEC B Series (which are a trade name, manufactured by Sekisui Chemical Co., Ltd.); polyurethane polyurea, such as BAYCUSAN Series (which are a trade name, manufactured by Covestro Japan Ltd.); hydroxyethyl cellulose, such as HEC DAICEL SE900, HEC DAICEL SE850, HEC DAICEL SE600, HEC DAICEL SE550, and HEC DAICEL SE400 (all of which are a trade name, manufactured by Daicel FineChem Ltd.); highly polymerized polyethylene glycol, such as POLYOX WSR N-12K, POLYOX WSR N-60K, and POLYOX WSR 301 (all of which are a trade name, manufactured by The Dow Chemical Company); PEO-27, PEO-18, PEO-15, and PEO-8 (all of which are a trade name of polyethylene oxide, manufactured by Sumitomo Seika Chemicals Co., Ltd.); polyvinylpyrrolidone, such as LUVISKOL K90, LUVISKOL K80, and LUVISKOL

K30 (all of which are a trade name, manufactured by BASF SE); and polyvinyl alcohol, such as GOHSENOL Series (which are a trade name, manufactured by Mitsubishi Chemical Corporation).

In the present invention, from the viewpoint of improving concealing performance and coloring performance, the dissolved amount of the polymer C in water is preferably less than 5 g in terms of the dissolved amount when the polymer C after drying at 105°C for 2 hours and reaching a constant weight is dissolved in 100 g of water at 25°C.

In the case where the polymer C is an anionic polymer, the aforementioned dissolved amount is the dissolved amount when the anionic group of the polymer C is neutralized with sodium hydroxide to an extent of 100%. In the case where the polymer C is a cationic polymer, the aforementioned dissolved amount is the dissolved amount when the cationic group of the polymer C is neutralized with hydrochloric acid to an extent of 100%.

From the viewpoint of improving concealing performance and coloring performance, the polymer C is preferably an amphipathic polymer which is insoluble in the solvent B but has affinity to the solvent B and which also has affinity to water, more preferably at least one selected from the group consisting of an ionic polymer and a nonionic polymer, still more preferably one that contains a polymer containing, as a monomer structural unit, at least one selected from the group consisting of an acid group-having monomer, a basic group-having monomer and a betaine monomer, yet still more preferably one that contains a polymer containing, as a monomer structural unit, at least one selected from the group consisting of an acid group-having monomer and a betaine monomer, even yet still more preferably, one that contains at least one selected from the group consisting of the anionic polymer CI and the betaine polymer CIII, and even still more preferably one that contains the betaine polymer CIII.

Also the polymer C is, from the viewpoint of improving waterproofness of cosmetic coating films while also improving concealing performance and coloring performance, preferably one that contains a polymer having a cationic group. The cationic group-having polymer is, from the same viewpoint as above, preferably at least one selected from the group consisting of a cationic polymer and a betaine polymer, more preferably a cationic polymer, still more preferably at least one selected from the group consisting of the cationic polymer CII-1 and the cationic silicone polymer CII-2, yet still more preferably the cationic polymer CII-1.

Further, for the polymer C, two or more kinds can be used as combined, from the viewpoint of improving waterproofness of cosmetic coating films while also improving concealing performance and coloring performance.

In the case where two or more kinds are used as combined for the polymer C, from the same viewpoint as above, the polymer C preferably contains at least two selected from the group consisting of the anionic polymer CI, the cationic polymer CII-1, the cationic silicone polymer CII-2 and the betaine polymer CIII, and is more preferably a combination of the anionic polymer CI and the cationic polymer CII-1, a combination of the anionic polymer CI and the cationic silicone polymer CII-2, a combination of the cationic polymer CII-1 and the cationic silicone polymer CII-2, a combination of the anionic polymer CI and the betaine polymer CIII, a combination of the cationic polymer CII-1 and the betaine polymer CIII, a combination of the cationic silicone polymer CII-2 and the betaine polymer CIII, or a combination of the anionic polymer CI, the cationic silicone polymer CII-2 and the betaine polymer CIII, still more preferably a combination of the anionic polymer CI, the cationic silicone polymer CII-2 and the betaine polymer CIII.

<Colorant>

With no limitation, the colorant for use in the present invention may be any one used in ordinary cosmetic materials, and includes an organic pigment, a hydrophobic dye (oil-soluble dye, disperse dye), and a water-soluble dye (e.g., acid dye, reactive dye, direct dye).

The hydrophobic dye is a dye whose solubility in 100 g of water (20°C) is preferably less than 6% by mass.

Specific examples of the colorant include organic pigments such as Red No. 201, Red. No. 202, Yellow No. 401, and Blue No. 404; lake pigments such as Red. No. 104, Red No. 230, Yellow No. 4, Yellow No. 5, and Blue No. 1; dyes such as Red No. 226, Acid Yellow 1, Acid Orange 7, Food Blue 2, and Acid Red 52; and those prepared by coating these pigments and dyes with a resin such as a polymethacrylic acid ester.

In the present invention, use of inorganic pigments is not impeded, so far as the advantageous effects of the present invention can be attained. Such inorganic pigments include white inorganic pigments such as titanium oxide and zinc oxide; non-white inorganic pigments such as yellow iron oxide, red iron oxide, black iron oxide, carbon black, ultramarine blue, Prussian blue, Prussian blue titanium oxide, black titanium oxide, chromium oxide, chromium hydroxide, and titanium/titanium oxide sintered product; and extender pigments such as calcium carbonate, silica and talc. In the case where the colorant contains an inorganic piment, the inorganic pigment is, from the viewpoint of concealing performance and coloring performance, preferably at least one selected from the group consisting of non-white inorganic pigments and extender pigments.

The colorant may be used alone or in combination of two or more thereof.

The hue of the colorant is, from the viewpoint of improving coloring performance, preferably a chromatic color such as yellow, magenta, cyan, blue, red, orange or green.

From the viewpoint of improving dispersibility in the cosmetic composition and from the viewpoint of waterproofness of cosmetic coating films, the colorant may be hydrophobized on the surface thereof. Also from the same viewpoint as above, in the case where the colorant contains an inorganic pigment, the surface of the inorganic pigment is preferably hydrophobized. The hydrophobization treatment is preferably one to be attained by the use of various hydrophobization treatment agents in ordinary cosmetic powders. Examples of the hydrophobization treatments include silicone treatment, fatty acid treatment, lauroyl lysine treatment, surfactant treatment, metal soap treatment, fluorine compound treatment, lecithin treatment, nylon treatment and polymer treatment.

The configuration of the colorant in the cosmetic composition of the present invention includes the configuration in which the colorant is dispersed in the cosmetic composition and the configuration in which the colorant is dissolved in the cosmetic composition. In particular, the configuration of the colorant is, from the viewpoint of improving concealing performance and coloring performance, and from the viewpoint of improving dispersibility in the cosmetic composition, preferably the configuration in which the colorant is dispersed in the cosmetic composition, more preferably the configuration in which the colorant is dispersed with a dispersant, or the configuration in which the colorant is self-dispersed without using a dispersant, and still more preferably the configuration in which the colorant is dispersed with a dispersant.

The self-dispersion configuration is preferably a self- dispersible pigment.

The self-dispersible pigment means a pigment onto a surface of which at least one functional group capable of imparting dispersibility is bonded either directly or through the other atom group to thereby render the pigment capable of being blended in a cosmetic composition without using a surfactant or a dispersant. The functional group capable of imparting dispersibility includes a anionic functional group such as a carboxy group or a sulfonic acid group, and also a polyethylene oxide group and a polypropylene oxide group. Specific examples of commercially available self-dispersible pigments include CAB-O-JET 200 series, CAB-O-JET 300 series and CAB-O-JET 400 series by Cabot Corporation.

The colorant is, from the viewpoint of coloration homogeneousness and from the viewpoint of improving concealing performance and coloring performance, preferably dispersed with a dispersant, dispersive polymer D.

In the present specification, "dispersive polymer" means a polymer that can disperse a colorant in a medium of a cosmetic composition.

The dispersive polymer D is, from the viewpoint of improving concealing performance and coloring performance, preferably soluble in any one of the solvent A and the solvent B and is insoluble in the other.

In the case where the dispersive polymer D is soluble in the solvent A and insoluble in the solvent B, the colorant in the system exists along with the dispersive polymer D and does not almost exist in the solvent B from viewpoint of the solubility of the dispersive polymer D in the solvent B, in the process of phase separation between the solvent A and the solvent B in forming a cosmetic coating film, and therefore in a cosmetic coating film, the colorant is contained mainly as a constituent component of the shell of the primary particles along with the polymer C.

On the other hand, in the case where the dispersive polymer D is insoluble in the solvent A and is soluble in the solvent B, the colorant is partitioned in the liquid drops of the phase-separated solvent B, in the process of phase separation between the solvent A and the solvent B in forming a cosmetic coating film, and therefore especially when the solvent B is volatile, the colorant exists in the hollows of the hollow primary particles in a cosmetic coating film.

The dispersive polymer D is, from the viewpoint of improving concealing performance and coloring performance, preferably soluble in the solvent A and is insoluble in the solvent B. The dispersive polymer D includes ionic polymers such as anionic polymers, cationic polymers and betaine polymers as well as nonionic polymers, such as those exemplified hereinabove for the polymer C. Above all, the dispersive polymer D is more preferably at least one selected from the group consisting of ionic polymers and nonionic polymers, still more preferably at least one selected from the group consisting of the above-mentioned anionic polymer C1, cationic polymer CII-1, cationic silicone polymer CII-2, betaine polymer CIII and nonionic polymers, yet still more preferably at least one selected from the group consisting of the anionic polymer CI and nonionic polymers.

The anionic polymer CI for use as the dispersive polymer D preferably contains a structural unit derived from an acid group-having monomer and a structural unit derived from a (meth)acrylic acid alkyl ester, more preferably contains a structural unit derived from an acid group-having monomer, a structural unit derived from a (meth)acrylic acid alkyl ester and a structural unit derived from an (N-alkyl)(meth)acrylamide, and is still more preferably a (meth)acrylic acid/(meth)acrylic acid alkyl ester /(N-alkyl)(meth)acrylamide copolymer, yet still more preferably an acrylic acid/acrylic acid alkyl ester /(N-alkyl)acrylamide copolymer.

The nonionic polymer for use as the dispersive polymer D is preferably at least one selected from the group consisting of a polyvinyl acetal and a polyurethane-polyurea, more preferably a polyvinyl acetal, still more preferably a polyvinyl butyral.

The acetalization degree of the polyvinyl butyral is, from the viewpoint of improving concealing performance and coloring performance, preferably 50 mol% or more, more preferably 55 mol% or more, still more preferably 60 mol% or more, and is preferably 80 mol% or less, more preferably 75 mol% or less, and still more preferably 70 mol% or less.

In the case where the colorant is dispersed with the dispersive polymer D, preferably, both the polymer C and the dispersive polymer D are at least one selected from the group consisting of an ionic polymer and a nonionic polymer.

The combination of the polymer C and the dispersive polymer D includes a combination of an ionic polymer of

the polymer C and an ionic polymer of the dispersive polymer D, a combination of an ionic polymer of the polymer C and a nonionic polymer of the dispersive polymer D, and a combination of a nonionic polymer of the polymer C and an ionic polymer of the dispersive polymer D.

The combination of the polymer C and the dispersive polymer D is preferably that the polymer C is at least one selected from the group consisting of the anionic polymer CI, the cationic polymer CII-1, the cationic silicone polymer CII-2, the betaine polymer CIII and a nonionic polymer, and the polymer D is at least one selected from the group consisting of the anionic polymer CI and a nonionic polymer, and more preferably that the polymer C is at least one selected from the group consisting of the anionic polymer CI, the cationic polymer CII-1, the cationic silicone polymer CII-2 and the betaine polymer CIII, and the polymer D is at least one selected from the group consisting of the anionic polymer CI and a nonionic polymer. In particular, the combination of the polymer C and the dispersive polymer D is, from the viewpoint of improving concealing performance and coloring performance, still more preferably that the polymer C is an anionic polymer or a betaine polymer, and the polymer D is an anionic polymer. From the viewpoint of improving waterproofness of cosmetic coating films, the combination of the polymer C and the dispersive polymer D is yet still more preferably that the polymer C is a cationic polymer or a betaine polymer and the polymer D is an anionic polymer. From the viewpoint of improving concealing performance and coloring performance, and from the viewpoint of waterproofness of cosmetic coating films, the combination of the polymer C and the dispersive polymer D is even yet still more preferably that the polymer C is a betaine polymer and the polymer D is an anionic polymer.

In the case where the colorant is dispersed with the dispersive polymer D, the volume-average particle size of the colorant particles dispersed with the polymer D is preferably 10 nm or more, more preferably 50 nm or more, still more preferably 100 nm or more, and is preferably 1,000 nm or less, more preferably 900 nm or less, still more preferably 500 nm or less, yet still more preferably 300 nm or less, and even yet still more preferably 200 nm or less. The volume-average particle size of the colorant particles is measured according to the method described in the section of Examples.

The cosmetic composition of the present invention may also contain, as an optional component, a component which is used for cosmetic compositions, such as a UV scattering agent, a UV absorbent, a fragrance, a beauty ingredient, a medicinal ingredient, a pH control agent, a moisturizer, an antioxidant, a disinfectant, and an antiseptic agent, unless the advantageous effects of the present invention are adversely affected thereby. Each of these may be used alone or in combination of two or more.

A viscosity at 20°C of the cosmetic composition of the present invention is preferably 1 mPa s or more, more preferably 2 mPa·s or more, and still more preferably 3 mPa·s or more, and is preferably 1,000 mPa s or less, more preferably 700 mPa·s or less, still more preferably 300 mPa·s or less, yet still more preferably 100 mPa·s or less, even yet still more preferably 50 mPa·s or less, even still more preferably 30 mPa·s or less, further more preferably 20 mPa·s or less, further more preferably 10 mPa s or less, and further more preferably 7 mPa s or less. The viscosity at 20°C of the cosmetic composition is measured by the method described in the section of Examples.

(Production of Cosmetic Composition)

The cosmetic composition can be obtained by mixing the solvent A, the solvent B, the polymer C, and a colorant, and optionally, the aforementioned optional component, followed by stirring. In the case where the colorant is dispersed with the dispersive polymer D, it is preferred that the colorant is mixed as a colorant dispersion containing colorant particles dispersed with the dispersive polymer D.

Although the mixing order of the solvent A, the solvent B, the polymer C, the colorant dispersion and the optional components is not particularly restricted, it is preferable to include a step of first mixing the solvent A and the polymer C to dissolve the polymer C in the solvent A, thereby obtaining a solution of the polymer C, and then adding and mixing the solvent B and the colorant dispersion to the foregoing solution. If desired, the aforementioned optional components may be further added and mixed.

The colorant dispersion can be prepared by dispersing a colorant and the dispersive polymer D, etc. by a known method. Specifically, the colorant dispersion is preferably prepared according to a method including a step of dispersing a colorant mixture containing a colorant, a dispersive polymer D and an organic solvent, though not necessarily limited to the method.

It is preferred that the organic solvent for use in producing the colorant dispersion has a high affinity to the dispersive polymer D and has good wettability to the colorant. The organic solvent is preferably ethanol or isopropanol, from the viewpoint of improving wettability to the colorant and adsorptivity of the dispersive polymer to the colorant, and also from the viewpoint of improving safety problems owing to the residual organic solvent in the cosmetic coating film after applicating the cosmetic composition.

As a means for applying a shear stress in the dispersing treatment of the production of the colorant dispersion, there may be used, for example, a kneading machine such as a roll mill, a kneader or an extruder, etc., a high-pressure homogenizer such as "Microfluidizer" (a trade name by Microfluidics Corporation), etc., and a media-assisted dispersing machine such as a paint shaker or a bead mill. Among these, from the viewpoint of reducing a particle size of the colorant,

the high-pressure homogenizer is preferably used.

In the case where the dispersing treatment is carried out using the high-pressure homogenizer, the particle size of the colorant can be adjusted to a desired value by controlling the treatment pressure and the number of passes through the homogenizer in the dispersing treatment.

The content of each component in the cosmetic composition of the present invention is as follows, from the viewpoint of improving concealing performance and coloring performance.

The content of the solvent A in the cosmetic composition of the present invention is preferably 30% by mass or more, more preferably 40% by mass or more, still more preferably 50% by mass or more, and is preferably 90% by mass or less, more preferably 80% by mass or less, still more preferably 70% by mass or less, yet still more preferably 60% by mass or less, even yet still more preferably 55% by mass or less.

The content of the solvent B in the cosmetic composition of the present invention is preferably 5% by mass or more, more preferably 7% by mass or more, still more preferably 10% by mass or more, yet still more preferably 13% by mass or more, and is preferably 40% by mass or less, more preferably 30% by mass or less, still more preferably 25% by mass or less, yet still more preferably 20% by mass or less, and even yet still more preferably 17% by mass or less.

The content of the polymer C in the cosmetic composition of the present invention is preferably 0.5% by mass or more, more preferably 1% by mass or more, and still more preferably 1.3% by mass or more, and is preferably 15% by mass or less, more preferably 10% by mass or less, still more preferably 5% by mass or less, yet still more preferably 3% by mass or less, and even yet still more preferably 2% by mass or less.

The total content of the solvent A and the solvent B in the cosmetic composition of the present invention is, from the viewpoint of improving concealing performance and coloring performance, preferably 50% by mass or more, more preferably 55% by mass or more, still more preferably 60% by mass or more, yet still more preferably 63% by mass or more, even yet still more preferably 65% by mass or more, and even still more preferably 67% by mass or more, and is preferably 85% by mass or less, more preferably 80% by mass or less, still more preferably 75% by mass or less, and yet still more preferably 70% by mass or less.

The ratio by mass of the content of the solvent A to the content of the solvent B [solvent A/solvent B] in the cosmetic composition of the present invention is, from the viewpoint of improving concealing performance and coloring performance, and from the viewpoint of promoting the developing speed of coloration, preferably 0.5 or more, more preferably 1 or more, still more preferably 1.5 or more, and yet still more preferably 2 or more, and is preferably 15 or less, more preferably 13 or less, still more preferably 11 or less, yet still more preferably 8 or less, even yet still more preferably 6 or less, even still more preferably 4 or less, and further more preferably 3 or less.

In the case where the polymer C contains a polymer that contains, as a structural unit, at least one selected from the group consisting of an acid group-having monomer, a basic group-having monomer and a betaine monomer, the content of the polymer that contains, as a structural unit, at least one selected from the group consisting of an acid group-having monomer, a basic group-having monomer and a betaine monomer in the polymer C is, from the viewpoint of improving concealing performance and coloring performance, preferably 60% by mass or more, more preferably 70% by mass or more, still more preferably 80% by mass or more, and yet still more preferably 90% by mass or less, and is preferably 100% by mass or less, and more preferably 100% by mass.

In the case where the polymer C contains, as a cationic group-having polymer, at least one selected from the group consisting of a cationic polymer and a betaine polymer, the content of the cationic group-having polymer in the polymer C is, from the viewpoint of improving waterproofness of cosmetic coating films while improving concealing performance and coloring performance, preferably 30% by mass or more, more preferably 40% by mass or more, still more preferably 50% by mass or more, and yet still more preferably 60% by mass or more, and is preferably 100% by mass or less, more preferably 90% by mass or less, and still more preferably 80% by mass or less.

In the case where two or more kinds are used as combined for the polymer C, it is preferred that the content of every polymer C in the cosmetic composition is adjusted so as to be the same amount.

The content of the colorant in the cosmetic composition of the present invention is, from the viewpoint of improving concealing performance and coloring performance, preferably 1% by mass or more, more preferably 2% by mass or more, still more preferably 3% by mass or more, and yet still more preferably 4% by mass or more, and is preferably 15% by mass or less, more preferably 10% by mass or less, and still more preferably 7% by mass or less.

In the case where the colorant is dispersed with the dispersive polymer D, the content of the dispersive polymer D in the cosmetic composition of the present invention is preferably 0.5% by mass or more, more preferably 1% by mass or more, and still more preferably 1.3% by mass or more, and is preferably 7% by mass or less, more preferably 5% by mass or less, still more preferably 3% by mass or less, and yet still more preferably 2% by mass or less.

In the case where the colorant is dispersed with the dispersive polymer D, the ratio by mass of the content of the colorant to the content of the dispersive polymer D [colorant/dispersive polymer D] in the cosmetic composition of the present invention is preferably 0.5 or more, more preferably 1 or more, and still more preferably 2 or more, and is preferably 5 or less, more preferably 4 or less, and still more preferably 3 or less.

In the case where the colorant is dispersed with the dispersive polymer D, the ratio by mass of the content of

the polymer C to the content of the dispersive polymer D [polymer C/dispersive polymer D] in the cosmetic composition of the present invention is preferably 0.3 or more, more preferably 0.5 or more, and still more preferably 0.7 or more, and is preferably 3 or less, more preferably 2 or less, still more preferably 1.5 or less, and yet still more preferably 1.3 or less.

The cosmetic composition of the present invention may also contain water unless the advantageous effects of the present invention are adversely affected thereby.

The content of water in the cosmetic composition of the present invention is, from the viewpoint of preventing phase separation of the solvent B in the cosmetic composition and improving concealing performance and coloring performance, preferably 5% by mass or less, more preferably less than 5%, still more preferably 4% by mass or less, yet still more preferably 3% by mass or less, even yet still more preferably 2% by mass or less, and even still more preferably 1% by mass or less, and is further more preferably, substantially 0% by mass, further more preferably 0% by mass.

The content of water in the cosmetic composition of the present invention means a total amount of water contained in the cosmetic composition, including the amount of water to be taken therein from the raw materials for the constituent components such as the solvent A, e.g., ethanol, as well as the amount of water to be mixed in the cosmetic composition by dewing in the production process thereof, in addition to the amount of water intentionally blended therein.

In the cosmetic composition of the present invention, use of inorganic pigments is not impeded, so far as the advantageous effects of the present invention as mentioned hereinabove can be attained, but the content of inorganic pigments in the cosmetic composition is preferably 5% by mass or less, more preferably 3% by mass or less, still more preferably 2% by mass or less, yet still more preferably 1% by mass or less, and even yet still more preferably 0% by mass.

The cosmetic composition of the present invention can be applied to skin (including lips of mouth), hair or nails, and is preferably applied by coating. With that, concealing performance and coloring performance can be given. Specifically, the cosmetic composition of the present invention is preferably used as a cosmetic composition for skin, a cosmetic composition for hair, or a cosmetic composition for nails, and is preferably used as a cosmetic composition for hair.

As a cosmetic composition for skin, the present invention is applicable to a base makeup cosmetic material, such as a makeup base, a foundation, and a concealer; a point makeup cosmetic material, such as a cheek rouge, an eyeshadow, a mascara, an eyeliner, an eyebrow cosmetic, an overcoat agent and a lipstick; a UV shielding cosmetic, such as a sunscreen milk lotion, and a sunscreen cream; a skin cleansing cosmetic, such as a facial cleanser and a cleansing cosmetic; and a basic cosmetic, such as a beauty essence, a beauty pack, and a massage cosmetic. Above all, from the viewpoint of improving concealing performance and coloring performance, the present invention is preferably applied to a base makeup cosmetic material, such as a makeup base and a foundation, and to a point makeup cosmetic material.

As a cosmetic composition for hair, the present invention is preferably applied to a hair dye such as a hair mascara and a hair color; a styling agent such as a hair wax, a hair spray, a hair mousse and a hair foam; and a hair growth agent.

As a cosmetic composition for nails, the present invention is preferably applied to a cosmetic for nail, such as a nail enamel and a nail gross.

As for a preparation form of the cosmetic composition of the present invention, it is possible to adapt for a solution form, a milk lotion form, a cream form, a gel form, a paste form, a solid form, a multilayered form, etc. Further, the cosmetic composition of the present invention can be applied as a spray agent, a sheet agent, or a mousse agent.

[Makeup Method]

Regarding the makeup method of the present invention, the cosmetic composition of the present invention is applied to skin, hair or nails for coating them with the cosmetic composition, according to an ordinary coating method for applying the cosmetic composition to skin, hair or nails under daily life temperature and humidity conditions.

The coating amount of the cosmetic composition is preferably 1 mg/cm$^2$ or more, more preferably 2 mg/cm$^2$ or more, and still more preferably 3 mg/cm$^2$ or more, and is preferably 10 mg/cm$^2$ or less, more preferably 7 mg/cm$^2$ or less, and still more preferably 5 mg/cm$^2$ or less.

The thickness of the coating film before drying is preferably 10 pm or more, more preferably 20 pm or more, and still more preferably 30 pm or more, and is preferably 300 pm or less, more preferably 200 pm or less, and still more preferably 150 pm or less.

In the makeup method of the present invention, from the viewpoint of improving concealing performance and coloring performance, pattern printing used in a printing system is preferably used for the coating method with the cosmetic composition. According to pattern printing, the coating amount and the coating region, etc. of the cosmetic composition can be controlled. Also by designing the pattern printing to be used, the makeup image to be applied can be previously planned. Further, by using the pattern printing, reproducibility of the cosmetic coating film having a complicated makeup image can be enhanced.

Examples of the pattern printing include on-demand printing such as printing by an inkjet method, printing by a dispenser method; and analogue printing such as screen printing, flexographic printing, gravure printing, offset printing, and the like. These printing methods may be appropriately selected and used according to the viscosity of the cosmetic composition, and the like.

The on-demand printing is a plateless printing method requiring no printing plate in which the printing is carried out in non-contact with the object to be printed, such as skin, hair or nails.

The analogue printing is a plate-based printing method requiring a printing plate in which the printing is carried out in contact with the object to be printed, such as skin, hair or nails.

Among these, the on-demand printing is capable of ejecting liquid drops of the cosmetic composition to apply a desired amount of the liquid drops to a desired region of the skin, hair or nails, and well controlling the mass of the liquid drops applied as measured per one drop thereof, the amount of the liquid drops applied per an unit area, the region to which the liquid drops are to be applied, and the like, so that it is possible to facilitate fine control of makeup images . From this viewpoint, the coating method with the cosmetic composition is more preferably a method of applying liquid drops of the cosmetic composition to skin, hair or nails by ejecting the cosmetic composition according to at least one selected from the group consisting of an inkjet method and a dispenser method, and still more preferably a method of applying liquid drops of the cosmetic composition to skin, hair or nails by ejecting the cosmetic composition according to an inkjet method.

The voltage applied to a inkjet head is, from the viewpoint of improving concealing performance and coloring performance, preferably 5 V or more, more preferably 10 V or more, and still more preferably 15 V or more, and is preferably 50 V or less, more preferably 45 V or less, and still more preferably 40 V or less.

The drive frequency of the inkjet head is, from the viewpoint of improving concealing performance and coloring performance, preferably 1 kHz or more, and more preferably 3 kHz or more, and is preferably 300 kHz or less, more preferably 150 kHz or less, still more preferably 90 kHz or less, and yet still more preferably 50 kHz or less.

The amount of the liquid drops to be ejected of the cosmetic composition is, from the viewpoint of improving concealing performance and coloring performance, preferably 0.01 pL or more, more preferably 0.1 pL or more, still more preferably 1 pL or more, and yet still more preferably 4 pL or more, and is preferably 50 pL or less, more preferably 40 pL or less, and still more preferably 35 pL or less, as calculated per one liquid drop ejected.

The impact density p of the liquid drops of the cosmetic composition in terms of the number of dots per square inch is preferably 10,000 or more, more preferably 30,000 or more, still more preferably 50,000 or more, and yet still more preferably 100,000 or more, and is preferably 3,000,000 or less, more preferably 1,000,000 or less, and still more preferably 500,000 or less. The impact density p of liquid drops is calculated from the product of the dot density (dpi) in the direction perpendicular to the printing direction and the dot density (dpi) in the printing direction.

In the present invention, preferably, after the cosmetic composition is applied to skin, hair or nails to form a coating film, the coating film is dried under an atmospheric pressure. In that manner, a cosmetic coating film excellent in concealing performance and coloring performance can be formed.

In the present invention, though drying of the coating film can be thoroughly performed by means of natural drying under daily life temperature and humidity conditions, it may be performed by means of blast drying, warm air drying, or the like from the viewpoint of quickly the drying. In the case of applying the cosmetic composition to skin, the coating film can be spontaneously dried at the skin temperature.

In the case of performing warm air drying, though the temperature in drying the coating film is not particularly restricted, it is preferably 40°C or higher, more preferably 50°C or higher, and still more preferably 55°C or higher, and is preferably 80°C or lower, more preferably 70°C or lower, and still more preferably 65°C or lower.

A drying time of the coating film is preferably 5 minutes or more, more preferably 7 minutes or more, and still more preferably 10 minutes or more, and is preferably 30 minutes or less, and more preferably 20 minutes or less.

Preferably from the viewpoint of improving concealing performance and coloring performance, the makeup method of the present invention includes, after a step of applying the cosmetic composition to skin, hair or nails (hereinafter also referred to as "step 1"), a step of applying liquid drops of a water-containing liquid E to the cosmetic composition applied to the skin, hair or nails (hereinafter also referred to as "step 2"), before drying the coating film of the cosmetic composition. In that manner, phase separation between the solvent A and the solvent B rapidly goes on to promote formation of primary particles of the solvent B coated with the polymer C, thereby enhancing concealing performance and coloring performance.

<Liquid E>

The liquid E in the present invention contains water, but may contain any other liquid than water.

The other liquid is preferably a monohydric alcohol having 1 or more and 4 or less carbon atoms, and example thereof include ethanol, n-propanol, isopropanol, and tert-butyl alcohol. Above all, from the viewpoint of improving concealing performance and coloring performance, preferred is at least one selected from the group consisting of ethanol,

n-propanol, isopropanol, and tert-butyl alcohol, and more preferred is ethanol.

The content of water in the liquid E is preferably 50% by mass or more, more preferably 60% by mass or more, still more preferably 70% by mass or more, yet still more preferably 80% by mass or more, even yet still more preferably 90% by mass or more, even still more preferably 95% by mass or more, further more preferably 97% by mass or more, and further more preferably 99% by mass or more, and is preferably 100% by mass or less, and more preferably 100% by mass.

The ethanol content as the other liquid in the liquid E is preferably 50% by mass or less, more preferably 40% by mass or less, still more preferably 30% by mass or less, yet still more preferably 20% by mass or less, even yet still more preferably 10% by mass or less, even still more preferably 5% by mass or less, further more preferably 3% by mass or less, and further more preferably 1% by mass or less.

The application method for liquid drops of the liquid E in the step 2 may be any method capable of applying fine liquid drops onto the surface of the cosmetic composition on skin, hair or nails, with no specific limitation, but from the viewpoint of improving concealing performance and coloring performance, preferably, a device of generating fine liquid drops of the water-containing liquid E is used. The device of generating fine liquid drops is not specifically limited, and examples thereof include a piezo-type device, a thermal device, a pressurizing device, a rotating device, a steam-type device, an ultrasonic device and an electrostatic device.

Among these, preferred is a method of using an atomizing device (sprayer) or the like to spray liquid drops or a method of applying liquid drops by pattern printing used in a printing system, and more preferred is a method of spray liquid drops with an atomizing device, or a method of ejecting liquid drops by an inkjet method.

An applied amount of liquid drops of the liquid E is, from the viewpoint of improving concealing performance and coloring performance, preferably 0.01 $mg/cm^2$ or more, more preferably 0.05 $mg/cm^2$ or more, and still more preferably 0.1 $mg/cm^2$ or more, and is preferably 10 $mg/cm^2$ or less, more preferably 7 $mg/cm^2$ or less, and still more preferably 5 $mg/cm^2$ or less.

An average size of liquid drops of the liquid E is, from the viewpoint of improving concealing performance and coloring performance, preferably 0.01 pm or more, more preferably 0.1 pm or more, and still more preferably 1 pm or more, and is preferably 50 pm or less, more preferably 30 pm or less, and still more preferably 10 pm or less.

The atomizing device includes a jet-type atomizing device, an ultrasonic atomizing device, and a mesh-type atomizing device.

The atomization performance of the atomizing device is preferably 0.01 mL/min or more, more preferably 0.1 mL/min or more, and still more preferably 0.3 mL/min or more, and is preferably 10 mL/min or less, more preferably 7 mL/min or less, and still more preferably 5 mL/min or less.

The atomization time with the atomizing device is preferably 1 second or more, and more preferably 3 seconds or more, and is preferably 30 seconds or less, more preferably 20 seconds or less, and still more preferably 10 seconds or less.

As the method of ejecting liquid drops in an inkjet method, employable is the same method as that in the application method for the cosmetic composition mentioned hereinabove.

Regarding application of liquid drops of the liquid E in the step 2, the liquid drops may be applied to the entire region where the cosmetic composition has been applied in the step 1 or in a part of the region.

The time distance between the step 1 and the step 2 is not specifically limited so far as the step 2 is started before the cosmetic composition on skin, hair or nails has dried, and is preferably 0.01 seconds or more, and more preferably 0.1 seconds or more, and is, from the viewpoint of improving concealing performance and coloring performance, preferably 10 seconds or less, and more preferably 5 seconds or less.

[Cosmetic Coating Film]

The average particle size of the primary particles contained in the cosmetic coating film formed according to the present invention is, from the viewpoint of increasing the strength of the cosmetic coating film and improving coloring performance, preferably 0.1 pm or more, and more preferably 0.2 pm or more, and is, from the viewpoint of improving concealing performance, preferably 5 pm or less, and more preferably 3 pm or less.

The average particle size of the primary particles contained in the cosmetic coating film can be measured by observing the formed cosmetic coating film with a scanning electron microscope, and then image-processing the resultant scanning electron micrograph with an image analysis software "Image J" (by the National Institutes of Health).

Specifically, using an optical microscope (a trade name: RH-2000, by Hirox-Japan Co., Ltd.), a photographic picture of the surface of the polymer coating film is taken at a magnification of 2,500, and then image-processed with an image analysis software (Image J, by the National Institutes of Health) to determine the average particle size of the primary particles contained in the coating film.

In taking a picture with the optical microscope, the luminance and the contrast are controlled in such a manner that the proportion of the pixels reaching the maximum luminance value is 1% or less and the average value of the

luminance falls within a range of 40% to 60% of the maximum luminance value, and the observational image is stored. Using an image transformation function of Image J, the observational image is transformed into a 8-bit grayscale image.

Next, scale setting is performed according to the magnification of the optical microscope. By the scale setting, the data of the particle size calculated by the next operation are converted into an actual size. As one example, in the case of a magnification of 2,500, a length of 1 mm is transformed to be 15,840 pixels.

Next, subtract background processing is performed. At that time, the rolling ball radius is set to be nearly the same size as that of the observed primary particle size. Specifically, in the case where primary particles having a diameter of around 3 pm account for a majority and where a length of 1 mm is 15,840 pixels, the rolling ball radius is 50 pixels. With that, the imaging noise component smaller than the primary particle size can be cut.

Next, using the threshold function of an image adjustment function of Image J, the region of the primary particles is processed for binarization.

In the present specification, "binarization processing" is a processing treatment in which, when the luminance value of the image is a designated value (threshold value) or more, it is white, and when the luminance value of the image is less than a designated value, it is black. In the image resulting from the observation, the shell of the primary particle is expressed to have a high luminance since the polymer abundance density therein is high, while the core part is expressed to have a low luminance. With that, it is possible to obtain an image having a clear shadow enough to measure the average particle size of the primary particles. The resultant binary image is further processed for "Analyze Particles" of Image J.

The particles partially cut at the edge of the image, as well as the particles having a major diameter of one-tenth of the primary particle size almost observed (in the above-mentioned setting, particles less than 5 pixels) and the particles having a circularity of less than 0.5 are excluded from the measuring objects, and an average value of the major diameters of at least 300 particles is taken here as an average particle size of primary particles.

Preferably, the cosmetic coating film formed according to the present invention contains secondary particles of accumulated primary particles. In the present invention, it is considered that, as a result of the evaporation of the solvent A and the surface alignment of the formed primary particles, there occur cell-like convective structures as regularly partitioned inside the coating film, so-called Benard cells, and owing to the Benard convection in each cell, the primary particles accumulate to form secondary particles. Especially when applied to skin, the cosmetic coating film containing such secondary particles can have a structure similar to the surface relief structure of actual skin, and therefore can form a cosmetic coating film that gives natural impressions.

EXAMPLES

In the following Synthesis Examples, Production Examples, Examples, and Comparative Examples, the terms "parts" and "%" are "parts by mass" and "% by mass", respectively unless otherwise indicated. The measurements of physical properties of polymers and others were performed by the following methods.

(1) Measurement of Weight Average Molecular Weight of Cationic Polymer CII-1

The measurement was performed using, as an eluent, a liquid in which phosphoric acid and lithium bromide were dissolved in concentrations of 60 mmol/L and 50 mmol/L, respectively in N,N-dimethylformamide by means of the gel permeation chromatography [GPC apparatus (HLC-8320GPC), manufactured by Tosoh Corporation, columns (TSKgel Super AWM-H, TSKgel Super AW3000, TSKgel guardcolumn Super AW-H), manufactured by Tosoh Corporation, flow rate: 1 mL/min] while using, as a standard substance, mono-dispersed polystyrene kits having already-known molecular weights [PStQuick B (F-550, F-80, F-10, F-1, A-1000) and PStQuick C (F-288, F-40, F-4, A-5000, A-500), all of which are manufactured by Tosoh Corporation].

As a measurement sample, one prepared by mixing 0.1 g of the cationic polymer CII-1 and 10 mL of the aforementioned eluent in a glass vial, stirring the mixture with a magnetic stirrer at 25°C for 10 hours, and filtering the resultant with a syringe filter (DISMIC-13HP PTFE, 0.2 pm, manufactured by Advantech Co., Ltd.) was used.

(2) Number Average Molecular Weight of Poly(N-propionylethyleneimine)

The measurement was performed using, as an eluent, 1 mmol/L of FARMIN DM20 (a trade name, manufactured by Kao Corporation)/chloroform by means of the gel permeation chromatography [measurement columns: two columns (K-804L), manufactured by Showa Denko K.K., connected in series, flow rate: 1 mL/min, column temperature: 40°C, detector: differential refractometer] while using, as a standard substance, polystyrene having an already-known molecular weight. 100 pL of the measurement sample having a concentration of 5 mg/mL was used.

(3) Measurement of Viscosity

The viscosity was measured with an E-type viscometer RE80, manufactured by Toki Sangyo Co., Ltd. at a rotation number of 100 rpm for a measurement time of 1 minute by using a standard rotor (1°34' × R24).
The measurement of the viscosity was performed at 20°C for hydrogenated polyisobutene, 25°C for the silicone oil, and 20°C for the cosmetic composition, respectively.

(4) Measurement of Volume-Average Particle Size of Colorant Particles

The volume-average particle size of colorant particles in a colorant dispersion was measured using a zeta potential/particle size measuring system "ELS-8000" (by Otsuka Electronics Co., Ltd.). A dispersion diluted with water so that the concentration of the particles to be analyzed could be about $5\times10^{-3}$% by mass was put into a measurement cell, and analyzed according to a cumulant analysis method in which a temperature of 25°C, a cumulative number of 100 times and a refractive index of water (1.333) as a refractive index of the dispersion medium were inputted.
Details of the respective components are as follows.

(Anionic Polymer CI)

ULTRAHOLD 8: An acrylic acid/acrylic acid alkyl ester/(N-alkyl) acrylamide copolymer (manufactured by BASF Japan Ltd., a trade name: ULTRAHOLD 8), powder with a solid component content of 100%
ULTRAHOLD STRONG: An acrylic acid/acrylic acid alkyl ester/(N-alkyl) acrylamide copolymer (manufactured by BASF Japan Ltd., a trade name: ULTRAHOLD STRONG), powder with a solid component content of 100%
ULTRAHOLD POWER-dry: A powder prepared by drying a solution of an acrylic acid/acrylic acid alkyl ester/(N-alkyl) acrylamide copolymer (solid component content: 32%) (manufactured by BASF Japan Ltd., a trade name:

ULTRAHOLD POWER)

(Cationic Polymer CII-1)

Cationic polymer 1: A copolymer obtained in the following Synthesis Example 1
Cationic polymer 2: A copolymer obtained in the following Synthesis Example 2

(Cationic Silicone Polymer CII-2)

Cationic silicone polymer 1: A poly(N-propionylethyleneimine)/dimethylpolysiloxane copolymer obtained in the following Synthesis Example 3
Cationic silicone polymer 2: A poly(N-propionylethyleneimine)/dimethylpolysiloxane copolymer obtained in the following Synthesis Example 4
Cationic silicone polymer 3: A poly(N-propionylethyleneimine)/dimethylpolysiloxane copolymer obtained in the following Synthesis Example 5

(Betaine Polymer CIII)

YUKA FORMER SM-dry: A powder prepared by drying an ethanol solution of an N-methacryloyloxyethyl-N,N-dimethylammonium-α-N-methylcarboxybetaine/methacrylic acid alkyl ester copolymer (solid component content: 30%) (manufactured by Mitsubishi Chemical Corporation, a trade name: YUKA FORMER SM)

(Nonionic Polymer)

Polyvinyl butyral: S-LEC BM-1 (manufactured by Sekisui Chemical Co., Ltd., a trade name, acetalization degree about 65 mol% (catalogue value)), solid content 100% powder
Polyurethane polyurea: A powder prepared by drying BAYCUSAN C2000 (manufactured by Covestro Japan Ltd.,

a trade name, an ethanol solution of polyurethane-64 with a solid component content of 40%)

(Solvent B)

[Hydrocarbon Oil]

PARLEAM 3: Hydrogenated polyisobutene (manufactured by NOF Corporation, a trade name: PARLEAM 3, boiling point: 179°C, Ra: 45, viscosity: 1.4 mPa·s)
PARLEAM 4: Hydrogenated polyisobutene (manufactured by NOF Corporation, a trade name: PARLEAM 4, boiling point: 262°C, Ra: 45, viscosity: 3.7 mPa·s)

[Silicone Oil]

KF-96A-1cs: Trisiloxane (manufactured by Shin-Etsu Chemical Co., Ltd., a trade name: KF-96A-1cs, boiling point: 153°C, Ra: 45, viscosity: 0.9 mPa·s)
TMF-1.5: Methyltrimethicone (manufactured by Shin-Etsu Chemical Co., Ltd., a trade name: TMF-1.5, boiling point: 191°C, Ra: 45, viscosity: 1.4 mPa·s)

Synthesis Example 1 (Synthesis of Cationic Polymer 1)

In a reaction vessel equipped with two dropping funnels 1 and 2, monomers and an organic solvent having a composition shown in the "Initially charged monomer solution" column of Table 1 were charged, followed by purging with a nitrogen gas.

Meanwhile, monomers and an organic solvent having a composition shown in the "Dropping monomer solution" column of Table 1 were mixed to prepare a dropping monomer solution; separately, a polymerization initiator (2,2'-azobis(2,4-dimethylvaleronitrile): manufactured by Fujifilm Wako Pure Chemical Corporation, a trade name: V-65) and an organic solvent shown in the "Polymerization initiator solution" column of Table 1 were mixed to prepare a polymerization initiator solution; and they were charged in the dropping funnels 1 and 2, respectively, followed by purging with a nitrogen gas.

The initially charged monomer solution in the reaction vessel was kept at 62°C in a nitrogen atmosphere while stirring, and the dropping monomer solution and the polymerization initiator solution were gradually dropped in the reaction vessel over 2 hours such that a proportion of the polymerization initiator to be dropped became constant relative to the monomers to be dropped.

After completion of dropping, the resultant was stirred for 1 hour while keeping at 62°C, and subsequently, 47 parts of acetone was added. The contents were further kept at 62°C while stirring and thermally aged for 4 hours.

Subsequently, the unreacted monomers and the polymerization initiator residue were removed from the reaction product by using an ultrafiltration membrane (manufactured by NGK Insulators, Ltd., a ceramic-made ultrafiltration membrane, a trade name: CEFILT, pore size: 10 nm), and the residue was then dried to obtain a cationic amphipathic polymer (hereinafter also referred to as "cationic polymer 1"). A weight average molecular weight of the obtained cationic polymer 1 was 130,000.

Synthesis Example 2 (Synthesis of Cationic Polymer 2)

In a reaction vessel equipped with two dropping funnels 1 and 2, monomers having a composition and an organic solvent shown in the "Initially charged monomer solution" column of Table 1 were charged, followed by purging with a nitrogen gas.

Meanwhile, monomers and an organic solvent having a composition shown in the "Dropping monomer solution" column of Table 1 were mixed to prepare a dropping monomer solution; and separately, a polymerization initiator (V-65) and an organic solvent shown in the "Polymerization initiator solution" column of Table 1 were mixed to prepare a polymerization initiator solution; and they were charged in the dropping funnels 1 and 2, respectively, followed by purging with a nitrogen gas.

The initially charged monomer solution in the reaction vessel was kept at 55°C in a nitrogen atmosphere while stirring, and the dropping monomer solution and the polymerization initiator solution were gradually dropped in the reaction vessel over 2 hours such that a proportion of the polymerization initiator to be dropped became constant relative

to the monomers to be dropped.

After completion of dropping, the contents were further kept at 55°C while stirring and thermally aged for 5 hours.

Subsequently, the unreacted monomers and the polymerization initiator residue were removed from the reaction product by using an ultrafiltration membrane (manufactured by NGK Insulators, Ltd., a ceramic-made ultrafiltration membrane, a trade name: CEFILT, pore size: 10 nm), and the residue was then dried to obtain a cationic amphipathic polymer (hereinafter also referred to as "cationic polymer 2"). A weight average molecular weight of the obtained cationic polymer 2 was 120,000.

Table 1

| | | Synthesis Example 1 | | | Synthesis Example 2 | | |
|---|---|---|---|---|---|---|---|
| | | Reaction vessel | Dropping funnel 1 | Dropping funnel 2 | Reaction vessel | Dropping funnel 1 | Dropping funnel 2 |
| | | Initially charged monomer solution | Dropping monomer solution | Polymerization initiator solution | Initially charged monomer solution | Dropping monomer solution | Polymerization initiator solution |
| Monomer composition (active ingredient) (parts) | DMAPAA *1 | 1.5 | 13.5 | | 1.0 | 9.0 | |
| | Ethyl acrylate | 2.5 | 22.5 | | 2.0 | 18.0 | |
| | t-BuAAm *2 | 5.0 | 45.0 | | 4.5 | 40.5 | |
| | NK ESTER M-90G *3 | 1.0 | 9.0 | | 2.5 | 22.5 | |
| Organic solvent (parts) | Acetone | 18.6 | 111.4 | 55.7 | | | |
| | Ethanol | | | | 10.0 | 60.0 | 30.0 |
| Polymerization initiator (parts) | V-65 *4 | | | 0.66 | | | 0.33 |
| Kind of cationic polymer CII-1 | | Cationic polymer 1 | | | Cationic polymer 2 | | |
| Weight average molecular weight of cationic polymer CII-1 | | 130,000 | | | 120,000 | | |

*1: N-[3-(Dimethylamino)propyl]acrylamide, manufactured by Sigma-Aldrich Co.
*2: N-tert-Butyl acrylamide, manufactured by Sigma-Aldrich Co.
*3: Methoxypolyethylene glycol monomethacrylate, manufactured by Shin-Nakamura Chemical Co., Ltd., a trade name: NK ESTER M-90G (ethylene oxide average addition molar number = 9, end: methyl group)
*4 2,2'-Azobis(2,4-dimethylvalelonitrile), manufactured by Fujifilm Wako Pure Chemical Corporation, a trade name: V-65

Synthesis Example 3 (Synthesis of Cationic Silicone Polymer 1)

12.9 g (0.13 mol) of 2-ethyl-2-oxazoline and 27.7 g of ethyl acetate were mixed, and the mixed liquid was dehydrated with 2.0 g of a molecular sieve (ZEOLUM A-4, manufactured by Tosoh Corporation) at 28°C for 15 hours. To the resulting dehydrated ethyl acetate solution of 2-ethyl-2-oxazoline, 0.77 g (0.005 mol) of diethyl sulfate was added, and the contents were heat-refluxed in a nitrogen atmosphere at 80°C for 8 hours, to obtain a terminal reactive poly(N-propionylethyleneimine) (number average molecular weight: 2,700) solution.
Separately, 100.0 g of side-chain primary aminopropyl-modified polydimethylsiloxane (manufactured by Shin-Etsu Chemical Co., Ltd., a trade name: KF-8015, weight average molecular weight: 100,000 (catalogue value), amine equivalent: 20,000) and 203.0 g of ethyl acetate were mixed, and the mixed liquid was dehydrated with 15.2 g of a molecular sieve at 28°C for 15 hours.
Subsequently, the above-obtained terminal reactive poly(N-propionylethyleneimine) solution was collectively added to the dehydrated side-chain primary aminopropyl-modified polydimethylsiloxane solution, and the contents were heat-refluxed at 80°C for 10 hours. The obtained reaction mixture was concentrated under reduced pressure to obtain a poly(N-propionylethyleneimine)/dimethylpolysiloxane copolymer (hereinafter also referred to as "cationic silicone polymer 1") as a white rubber-like solid (108 g). A weight average molecular weight of the cationic silicone polymer 1 was 115,000 (calculated value), and a mass ratio [{content of organopolysiloxane segment (x)}/[total content of {organopolysiloxane segment (x)} and {poly(N-acylalkyleneimine) segment (y)}]] was 0.87.

Synthesis Example 4 (Synthesis of Cationic Silicone Polymer 2)

53.3 g (0.54 mol) of 2-ethyl-2-oxazoline and 127.5 g of ethyl acetate were mixed, and the mixed liquid was dehydrated with 9.0 g of a molecular sieve (ZEOLUM A-4, manufactured by Tosoh Corporation) for 15 hours. To the resulting dehydrated ethyl acetate solution of 2-ethyl-2-oxazoline, 9.48 g (0.061 mol) of diethyl sulfate was added, and the contents were heat-refluxed in a nitrogen atmosphere at 80°C for 8 hours, to obtain a terminal reactive poly(N-propionylethyleneimine) (number average molecular weight: 1,300) solution.
Separately, 153.7 g of side-chain primary aminopropyl-modified polydimethylsiloxane (manufactured by Shin-Etsu Chemical Co., Ltd., a trade name: KF-8003, weight average molecular weight: 40,000 (catalogue value), amine equivalent: 2,000) and 312.1 g of ethyl acetate were mixed, and the mixed liquid was dehydrated with 23.3 g of a molecular sieve at 28°C for 15 hours.
Subsequently, the above-obtained terminal reactive poly(N-propionylethyleneimine) solution was collectively added to the dehydrated side-chain primary aminopropyl-modified polydimethylsiloxane solution, and the contents were heat-refluxed at 80°C for 10 hours. The reaction mixture was concentrated under reduced pressure to obtain a poly(N-propionylethyleneimine)/dimethylpolysiloxane copolymer (hereinafter also referred to as "cationic silicone polymer 2") as a pale yellow rubber-like solid (200 g). A weight average molecular weight of the cationic silicone polymer 2 was 56,000 (calculated value), and a mass ratio [{content of organopolysiloxane segment (x)}/[total content of {organopolysiloxane segment (x)} and {poly(N-acylalkyleneimine) segment (y)}]] was 0.71.

Synthesis Example 5 (Synthesis of Cationic Silicone Polymer 3)

73.7 g (0.74 mol) of 2-ethyl-2-oxazoline and 156.0 g of ethyl acetate were mixed, and the resultant mixed liquid was dehydrated with 12.0 g of a molecular sieve (ZEOLUM A-4, manufactured by Tosoh Corporation) at 28°C for 15 hours. To the resulting dehydrated ethyl acetate solution of 2-ethyl-2-oxazoline, 2.16 g (0.014 mol) of diethyl sulfate was added, and the contents were heat-refluxed in a nitrogen atmosphere at 80°C for 8 hours, to obtain a terminal reactive poly(N-propionylethyleneimine) (number average molecular weight: 6,000) solution.
Separately, 70.0 g of side-chain primary aminopropyl-modified polydimethylsiloxane (KF-864, manufactured by Shin-Etsu Silicone Co., Ltd., weight average molecular weight: 50,000 (catalogue value), amine equivalent: 3,800) and 140.0 g of ethyl acetate were mixed, and the mixed liquid was dehydrated with 15.0 g of a molecular sieve at 28°C for 15 hours.
Subsequently, the above-obtained terminal reactive poly(N-propionylethyleneimine) solution was collectively added to the above-dehydrated side-chain primary aminopropyl-modified polydimethylsiloxane solution, and the contents were heat-refluxed at 80°C for 10 hours. The reaction mixture was concentrated under reduced pressure to obtain a poly(N-propionylethyleneimine)/dimethylpolysiloxane copolymer (hereinafter also referred to as "cationic silicone polymer 3") as a white rubber-like solid (135 g). A weight average molecular weight of the cationic silicone polymer 3 was 100,000 (calculated value), and a mass ratio [{content of organopolysiloxane segment (x)}/[total content of {organopolysiloxane segment (x)} and {poly(N-acylalkyleneimine) segment (y)}]] was 0.50.

Production Example 1-1 (Production of colorant dispersion 1)

As the dispersive polymer D (anionic polymer CI), 250 g of an ethanol solution of an anionic acrylic polymer "Plas Size L-9909U" (by Goo Chemical Co., Ltd., acid value: 50 mg KOH/g, 100% neutralization, neutralizing agent: 2-amino-2-methyl-1-propanol, ethanol solution having a solid concentration of 40% by mass) was put into a sealable and temperature-controllable glass jacket, and with stirring at a jacket temperature of 15°C and under the condition of 1,400 rpm using a high-speed disperser "T. K. Robomix" (by PRIMIX Corporation) (stirring part: Homodisper 2.5 Model (blade diameter 40 mm), 300 g of a colorant, Red No. 226 K (by Kishi Kasei Co., Ltd., red dye (C. I. Vat Red 1) was added thereto, and further stirred at a jacket temperature of 15°C and under the condition of 2,000 rpm for 1 hour to allow the colorant to blend with the anionic acrylic polymer solution.

Next, while the jacket temperature was kept at 15°C, the rotation speed was changed to 8,000 rpm, and 1450 g of a first grade ethanol was added, and stirred for 3 hours to give a colorant mixture 1 (solid concentration 20% by mass).

Using a Microfluidizer (by Microfluidics Corporation, Model: M-140K), under a pressure of 150 MPa, the resultant colorant mixture 1 was treated for dispersion in a total of 10 passes to give a colorant dispersion 1 having a solid concentration of 20% by mass.

The amount of dissolution of the anionic acrylic polymer used as the dispersive polymer D (anionic polymer CI) in 100 g of the solvent B used in Examples and Comparative Examples given hereinunder was less than 5 g, and the amount of dissolution thereof in 100 g of the solvent A was 5 g or more.

Production Example 1-2 (Production of colorant dispersion 2)

As the dispersive polymer D (nonionic polymer), 100 g of a nonionic polyvinyl butyral "S-LEC BM-1" (by Sekisui Chemical Co., Ltd., effective content 100% powder) was dissolved in 900 g of a first grade ethanol in a sealable and temperature-controllable glass jacket to give an ethanol solution of nonionic polyvinyl butyral having a solid concentration of 10% by mass. Next, in the same manner as in Production Example 1-1 except that the ethanol solution of nonionic polyvinyl butyral was used in place of the ethanol solution of anionic acrylic polymer and that the colorant mixture was changed to have the formulation composition as in Table 2, a colorant dispersion 2 was produced.

The amount of dissolution of the nonionic polyvinyl butyral used as the dispersive polymer D (nonionic polymer) in 100 g of the solvent B used in Examples and Comparative Examples given hereinunder was less than 5 g, and the amount of dissolution thereof in 100 g of the solvent A was 5 g or more.

Table 2

| | | Production Example | |
|---|---|---|---|
| | | 1-1 | 1-2 |
| Kind of Colorant Dispersion | | 1 | 2 |
| Kind of Colorant | | Red No. 226 K | Red No. 226 K |
| Formulation Composition of Colorant Mixture (g) | Colorant | 300 | 300 |
| | Anionic Acryl Polymer Solution (solid concentration 40% by mass ethanol solution) | 250 | 0 |
| | Nonionic Polyvinyl Butyral Solution (solid concentration 10% by mass ethanol solution | 0 | 1000 |
| | Ethanol | 1450 | 700 |
| Ratio by mass of Colorant Mixture [Colorant/Dispersive Polymer D] | | 3 | 3 |
| Ratio by mass of Colorant Mixture [(colorant + Dispersive Polymer D)/Total Amount] | | 0.20 | 0.20 |
| Condition of Dispersion Treatment for Colorant Mixture | | 150 MPa, 10 passes | 150 MPa, 10 passes |
| Volume-Average Particle Size (nm) of Colorant Particles | | 178 | 190 |

Examples 1-1 to 1-5 and Comparative Examples 1-1 to 1-7

(Production of Cosmetic Composition)

1.5 parts of YUKA FORMER SM-dry as the polymer C (betaine polymer CIII) was dissolved in 58.5 parts of the solvent A described in Table 3; after confirming that the solution was transparent and free from a floating material and a precipitate, 10 parts of the solvent B described in Table 3 was added, and then 30 parts of the colorant dispersion 1 was added; the contents were stirred and homogenized; and the resultant mixture was filtered to give cosmetic compositions X-1 to X-5 and XC-1 to XC-7. The viscosity at 20°C of the obtained cosmetic compositions is shown in Table 3.

As for the membrane filter used for filtration of the mixture, from the viewpoint of solvent resistance of the filter itself, a cellulose acetate syringe filter having a pore size of 1.20 pm (by Sartorius Inc.) was used for the production of cosmetic compositions X-1 to X-5 and XC-1 to XC-2 and XC-7, and a hydrophilized PTFE syringe filter having a pore size of 0.45 pm (by Advantech Co., Ltd.) was used for the production of cosmetic compositions XC-3 to XC-6.

The dissolved amount of the polymer C (YUKA FORMER SM-dry) used in Example 1-1 in 100 g of the solvent A (anhydrous ethanol) was 50 g, and the dissolved amount thereof in 100 g of the solvent B (PARLEAM 3) was 0.3 g. In addition, the dissolved amount of the polymer C (YUKA FORMER SM-dry) used in Examples 1-2 to 1-5 in 100 g of the solvent B was less than 5 g, and the dissolved amount thereof in 100 g of the solvent A was 5 g or more.

(Makeup Method (production of cosmetic coating film))

After the inside of an ink cartridge "TK403 Black-CS Cartridge" (by Kishu Giken Kogyo Co., Ltd.) was washed with ion exchanged water and ethanol, the ink cartridge was filled with any of the cosmetic compositions of Examples and Comparative Examples, and mounted on an handy inkjet printer (by Kishu Giken Kogyo Co., Ltd., a trade name: KGKJET HQ1000H) that had been so modified as to be drivable under a printing condition of 600 dpi.

Next, in an environmental chamber, the temperature and humidity of which were controlled at a temperature of 25°C and a humidity of 50%, a black PET film "Lumirror S10" (by Toray Corporation) as an evaluation substrate was fixed on a horizontal bed, and using the handy inkjet printer, the evaluation substrate was solid-printed with the cosmetic composition from the vertical upper surface direction.

For the printing, the resolution was 600 dpi in length and 600 dpi in width, and a solid image having a size of 12.7 mm in length and 50.8 mm in width (dot density p: 360,000 (dot/square inch) was used.

Tracking the inkjet head of the handy inkjet printer immediately after the printing, fine liquid drops of an ion exchanged water as the liquid E were sprayed over the surface of the coating film, using an ultrasonic nebulizer "COMFORT OASIS" (by Shin-Ei Industries, Inc., Model: KU-200, average size of liquid drops: 1 to 5 pm), and in 1 second after the completion of the solid image printing, the power source of the ultrasonic nebulizer was cut off to stop spraying with the liquid drops.

Next, after left in a temperature and humidity-controlled environmental chamber at a temperature of 25°C and a humidity of 50% for 30 minutes, prints of cosmetic coating films 1-1 to 1-5 and 1-C1 to 1-C7 each formed from the corresponding cosmetic compositions were obtained.

(Evaluation of Concealing Performance and Coloring Performance)

The magenta image density (hereinafter also referred to as "magenta density") and a black image density (hereinafter also referred to as "black density") of the prints obtained in Examples and Comparative Examples were measured using the measurement apparatus mentioned below under the measurement condition also mentioned below. The results are shown in Table 3.

Measurement Apparatus: Spectral Colorimeter/Densitometer "SpectroEye" (by X-Rite Inc.)
Measurement Condition: Light Source D65, Observation Visual Field 2 degrees, Density Standard DIN, White Base "Abs", Internal Filter "No"

The black PET film used as the evaluation substrate was measured, and the found data of the black density and the magenta density were both 1.90.

Regarding evaluation of concealing performance and coloring performance, it is desirable that the black density is low and the magenta density is high from the viewpoint of color vividness. When the concealing performance of the cosmetic coating film is insufficient, the black density is high and the magenta coloration could be difficultly developed. From the viewpoint, the density difference between the magenta density and the black density (magenta density - black density) is preferably 0.8 or more, more preferably 0.9 or more, and still more preferably 1.0 or more.

In Examples and Comparative Examples, for evaluation of concealing performance and coloring performance, the black PET film was used as a substitute for skin, hair and nails, and it was confirmed that, also in the case of applying to skin, hair and nails, the effect can be evaluated on the same criteria.

Table 3

| | | | | | Example | | | Comparative Example | | | | Example | | Comparative Example | | |
|---|---|---|---|---|---|---|---|---|---|---|---|---|---|---|---|---|
| | | | | | 1-1 | 1-2 | 1-3 | 1-1 | 1-2 | 1-3 | 1-4 | 1-4 | 1-5 | 1-5 | 1-6 | 1-7 |
| Cosmetic Composition No. | | | | | X-1 | X-2 | X-3 | XC-1 | XC-2 | XC-3 | XC-4 | X-4 | X-5 | XC-5 | XC-6 | XC-7 |
| Composition (parts) | Kind | | Boiling Point (°C) | Ra | | | | | | | | | | | | |
| | Solvent A | Anhydrous Ethanol | 78 | 24 | 58.5 | | | | | | | 58.5 | 58.5 | 58.5 | 58.5 | 68.5 |
| | | N-propanol | 97 | 27 | | 58.5 | | | | | | | | | | |
| | | Anhydrous Isopropanol | 82 | 28 | | | 58.5 | | | | | | | | | |
| | Solvent A' | Butanol | 118 | 28 | | | | 58.5 | | | | | | | | |
| | | Pentanol | 138 | 30 | | | | | 58.5 | | | | | | | |
| | | Chloroform | 61 | 39 | | | | | | 58.5 | | | | | | |
| | | Toluene | 111 | 43 | | | | | | | 58.5 | | | | | |
| | Kind | | Boiling Point (°C) | Ra | | | | | | | | | | | | |
| | Solvent B | PARLEAM 3 | 179 | 45 | 10 | 10 | 10 | 10 | 10 | 10 | 10 | | | | | |
| | | PARLEAM 4 | 262 | 45 | | | | | | | | 10 | | | | |
| | | Isododecane | 177 | 45 | | | | | | | | | 10 | | | |
| | Solvent B' | Hexane | 69 | 45 | | | | | | | | | | 10 | | |
| | | Dimethylformamide | 153 | 31 | | | | | | | | | | | 10 | |
| | Polymer C | YUKA FORMER SM-dry | | | 1.5 | 1.5 | 1.5 | 1.5 | 1.5 | 1.5 | 1.5 | 1.5 | 1.5 | 1.5 | 1.5 | 1.5 |
| | Colorant Dispersion | Colorant Dispersion 1 | | | 30 | 30 | 30 | 30 | 30 | 30 | 30 | 30 | 30 | 30 | 30 | 30 |
| Viscosity of Cosmetic Composition (mPa·s) | | | | | 3.8 | 4.2 | 4.0 | 5.1 | 5.5 | 3.1 | 3.1 | 3.9 | 3.8 | 2.7 | 3.4 | 3.1 |
| Evaluation | Cosmetic Coating Film No. | | | | 1-1 | 1-2 | 1-3 | 1-C1 | 1-C2 | 1-C3 | 1-C4 | 1-4 | 1-5 | 1-C5 | 1-C6 | 1-C7 |
| | Magenta Density | | | | 1.70 | 1.70 | 1.70 | 1.69 | 1.70 | 1.68 | 1.68 | 1.70 | 1.69 | 1.70 | 1.69 | 1.70 |
| | Black Density | | | | 0.53 | 0.87 | 0.74 | 1.62 | 1.63 | 1.53 | 1.57 | 0.74 | 0.72 | 1.58 | 1.57 | 1.64 |
| | Density Difference (magenta density – black density) | | | | 1.17 | 0.83 | 0.96 | 0.07 | 0.07 | 0.15 | 0.11 | 0.96 | 0.97 | 0.12 | 0.12 | 0.06 |

From Table 3, it was confirmed that Examples 1-1 to 1-5 have a low black density and are excellent in concealing performance, and have a high magenta density and a density difference between the magenta density and the black density of 0.8 or more and are excellent in coloring performance.

On the other hand, it was confirmed that Comparative Examples 1-1 to 1-7 have a high black density and are poor in concealing performance, and have a small density difference between the magenta density and the black density and are poor in coloring performance.

Examples 2-1 to 2-30

(Production of Cosmetic Composition)

The polymer C (anionic polymer CI, cationic polymer CII-1, cationic silicone polymer CII-2, betaine polymer CIII, or nonionic polymer) shown in Table 4 and Table 5 was dissolved in anhydrous ethanol (boiling point 78°C, Ra 24) as the solvent A; after confirming that the solution was transparent and free from a floating material and a precipitate, PARLEAM 3 (boiling point 179°C, Ra 45) was added as the solvent B; then the colorant dispersion was added and the contents were stirred and homogenized; and the resultant mixture was filtered through a cellulose acetate syringe filter having a pore size of 1.20 pm (by Sartorius Inc.). There were thus obtained cosmetic compositions Y-1 to Y-30.

The dissolved amount of the polymer C used in Examples 2-1 to 2-30 in 100 g of the solvent B (PARLEAM 3) was less than 5 g, and the dissolved amount thereof in 100 g of the solvent A (anhydrous ethanol) was 5 g or more.

(Makeup Method (production of cosmetic coating film) and Evaluation of Concealing Performance and Coloring Performance)

According to the same method as in Example 1-1, prints having a cosmetic coating film 2-1 to 2-30 were obtained and the image density thereof was measured to evaluate concealing performance and coloring performance. The results are shown in Table 4 and Table 5.

(Evaluation of Waterproofness)

The resultant print was put on a horizontal flat plate, 0.1 g of ion-exchanged water was dropped onto the cosmetic coating film via a dropper, and left in a temperature and humidity-controlled environmental chamber at a

temperature of 25°C and a humidity of 50% for 1 minute. Next, while the part onto which ion-exchanged water had dropped was rubbed 5 times with a cotton swab having a diameter of 5 mm and wetted with ion-exchanged water, the surface condition of the cosmetic coating film was observed, and then this was dried with a hot air drier until the water drops on the part on which the water drops had dropped dried and disappeared. Next, the surface condition of the rubbed cosmetic coating film was further observed, and evaluated according to the following 5-rank evaluation criteria. The results are shown in Table 4 and Table 5.

(Evaluation Criteria)

5: While rubbed in a wet state, no change appeared on the surface condition of the cosmetic coating film, and there occurred no color fading.

4: While rubbed in a wet state, some color change appeared in the cosmetic coating film, but after drying, the surface was restored to the original condition with no color fading. No problem for practical use.

3: While rubbed in a wet state, some color change and wrinkles appeared in the cosmetic coating film, but after drying, the surface was restored to the original condition with no color fading. No problem for practical use.

2: While rubbed in a wet state, distinct color fading appeared in the cosmetic coating film, and even after drying, color fading was obviously recognized.

1: While rubbed in a wet state, the cosmetic coating film broke, and after drying, this greatly differed from the original surface condition.

Table 4

| | | | | Example | | | | | | | | | | |
|---|---|---|---|---|---|---|---|---|---|---|---|---|---|---|
| | | | | 2-1 | 2-2 | 2-3 | 2-4 | 2-5 | 2-6 | 2-7 | 2-8 | 2-9 | 2-10 | 2-11 |
| Cosmetic Composition No. | | | | Y-1 | Y-2 | Y-3 | Y-4 | Y-5 | Y-6 | Y-7 | Y-8 | Y-9 | Y-10 | Y-11 |
| Composition (parts) | Solvent A | | Anhydrous Ethanol | 48.5 | 48.5 | 48.5 | 48.5 | 48.5 | 48.5 | 48.5 | 48.5 | 48.5 | 48.5 | 48.5 |
| | Solvent B | | PARLEAM 3 | 20 | 20 | 20 | 20 | 20 | 20 | 20 | 20 | 20 | 20 | 20 |
| | Polymer C | Anionic Polymer CI | ULTRAHOLD 8 | | 1.5 | | | | | 0.75 | | | | |
| | | | ULTRAHOLD Strong | | | 1.5 | | | | | 0.75 | | | |
| | | | ULTRAHOLD Power-dry | | | | 1.5 | | | | | 0.75 | | |
| | | Cationic Polymer CII-1 | Cationic Polymer 1 | | | | | 1.5 | | | | | 0.75 | |
| | | | Cationic Polymer 2 | | | | | | 1.5 | | | | | 0.75 |
| | | Betaine Polymer CIII | YUKA FORMER SM-dry | 1.5 | | | | | | 0.75 | 0.75 | 0.75 | 0.75 | 0.75 |
| | Colorant Dispersion | | Colorant Dispersion 1 | 30 | 30 | 30 | 30 | 30 | 30 | 30 | 30 | 30 | | |
| | | | Colorant Dispersion 2 | | | | | | | | | | 30 | 30 |
| Viscosity of Cosmetic Composition (mPa·s) | | | | 3.8 | 3.6 | 3.5 | 3.5 | 4.1 | 4.0 | 3.7 | 3.5 | 3.5 | 4.3 | 4.4 |
| Evaluation | Cosmetic Coating Film No. | | | 2-1 | 2-2 | 2-3 | 2-4 | 2-5 | 2-6 | 2-7 | 2-8 | 2-9 | 2-10 | 2-11 |
| | Magenta Density | | | 1.71 | 1.75 | 1.74 | 1.74 | 1.61 | 1.61 | 1.70 | 1.70 | 1.70 | 1.75 | 1.75 |
| | Black Density | | | 0.40 | 0.51 | 0.51 | 0.52 | 0.77 | 0.80 | 0.46 | 0.50 | 0.51 | 0.50 | 0.54 |
| | Density Difference (magenta density – black density) | | | 1.31 | 1.24 | 1.23 | 1.22 | 0.84 | 0.81 | 1.24 | 1.20 | 1.19 | 1.25 | 1.21 |
| | Waterproofness | | | 4 | 3 | 3 | 3 | 5 | 5 | 4 | 4 | 4 | 4 | 4 |

Table 5

| | | | Example | | | | | | | | | | | | | | | | | | |
|---|---|---|---|---|---|---|---|---|---|---|---|---|---|---|---|---|---|---|---|---|---|
| | | | 2-12 | 2-13 | 2-14 | 2-15 | 2-16 | 2-17 | 2-18 | 2-19 | 2-20 | 2-21 | 2-22 | 2-23 | 2-24 | 2-25 | 2-26 | 2-27 | 2-28 | 2-29 | 2-30 |
| Cosmetic Composition No. | | | Y-12 | Y-13 | Y-14 | Y-15 | Y-16 | Y-17 | Y-18 | Y-19 | Y-20 | Y-21 | Y-22 | Y-23 | Y-24 | Y-25 | Y-26 | Y-27 | Y-28 | Y-29 | Y-30 |
| Composition (parts) | Solvent A | Anhydrous Ethanol | 48.5 | 48.5 | 48.5 | 48.5 | 48.5 | 48.5 | 48.5 | 48.5 | 48.5 | 48.5 | 48.5 | 48.5 | 48.5 | 48.5 | 48.5 | 48.5 | 48.5 | 48.5 | 48.5 |
| | Solvent B | PARLEAM 3 | 20 | 20 | 20 | 20 | 20 | 20 | 20 | 20 | 20 | 20 | 20 | 20 | 20 | 20 | 20 | 20 | 20 | 20 | 20 |
| | Polymer C: Anionic Polymer CI | ULTRAHOLD 8 | 0.75 | 0.75 | 0.75 | 0.75 | 0.75 | | | | | | | | | | | | 0.5 | | |
| | | ULTRAHOLD Strong | 0.75 | | | | | 0.75 | 0.75 | 0.75 | 0.75 | | | | | | | | | | |
| | | ULTRAHOLD Power-dry | | 0.75 | | | | 0.75 | | | | 0.75 | 0.75 | | | | | | | | |
| | Polymer C: Cationic Polymer CII-1 | Cationic Polymer 1 | | | 0.75 | | | | 0.75 | | | | | 0.75 | 0.75 | | | | | | |
| | | Cationic Polymer 2 | | | | 0.75 | | | | | | | | | | 0.75 | 0.75 | 0.75 | | | |
| | Polymer C: Cationic Silicone Polymer CII-2 | Cationic Silicone Polymer 1 | | | | | | | | 0.75 | | | | | | 0.75 | | | | | |
| | | Cationic Silicone Polymer 2 | | | | | | | | | | 0.75 | | 0.75 | | | 0.75 | | | | |
| | | Cationic Silicone Polymer 3 | | | | | 0.75 | | | | 0.75 | | 0.75 | | 0.75 | | | 0.75 | 0.5 | | |
| | Polymer C: Betaine Polymer CIII | YUKA FORMER SM-dry | | | | | | | | | | | | | | | | | 0.5 | | |
| | Polymer C: Nonionic Polymer | Polyvinyl Butyral | | | | | | | | | | | | | | | | | | 1.5 | |
| | | Polyurethane Polyurea | | | | | | | | | | | | | | | | | | | 1.5 |
| | Colorant Dispersion | Colorant Dispersion 1 | 30 | 30 | | | | 30 | | | | | | | | | | | | 30 | 30 |
| | | Colorant Dispersion 2 | | | 30 | 30 | 30 | | 30 | 30 | 30 | 30 | 30 | 30 | 30 | 30 | 30 | 30 | 30 | | |
| Viscosity of Cosmetic Composition (mPa s) | | | 3.5 | 3.5 | 5.0 | 4.9 | 4.8 | 3.6 | 4.9 | 4.5 | 4.4 | 4.5 | 4.4 | 4.2 | 4.0 | 4.1 | 4.0 | 4.1 | 4.3 | 3.6 | 3.8 |
| Evaluation | Cosmetic Coating Film No. | | 2-12 | 2-13 | 2-14 | 2-15 | 2-16 | 2-17 | 2-18 | 2-19 | 2-20 | 2-21 | 2-22 | 2-23 | 2-24 | 2-25 | 2-26 | 2-27 | 2-28 | 2-29 | 2-30 |
| | Magenta Density | | 1.70 | 1.69 | 1.70 | 1.70 | 1.69 | 1.68 | 1.69 | 1.70 | 1.70 | 1.70 | 1.69 | 1.70 | 1.70 | 1.70 | 1.69 | 1.69 | 1.70 | 1.70 | 1.70 |
| | Black Density | | 0.64 | 0.63 | 0.77 | 0.75 | 0.59 | 0.65 | 0.73 | 0.60 | 0.61 | 0.62 | 0.59 | 0.67 | 0.68 | 0.66 | 0.69 | 0.70 | 0.51 | 0.65 | 0.77 |
| | Density Difference (magenta density – black density) | | 1.06 | 1.06 | 0.93 | 0.95 | 1.10 | 1.03 | 0.96 | 1.10 | 1.09 | 1.08 | 1.10 | 1.03 | 1.02 | 1.04 | 1.00 | 0.99 | 1.19 | 1.05 | 0.93 |
| | Waterproofness | | 3 | 3 | 5 | 5 | 5 | 3 | 5 | 5 | 5 | 5 | 5 | 4 | 4 | 4 | 4 | 4 | 5 | 3 | 3 |

From Table 4 and Table 5, it was confirmed that Examples 2-1 to 2-30 have a low black density and are excellent in concealing performance, and have a high magenta density and a density difference between the magenta density and the black density of 0.8 or more and are excellent in coloring performance, and in addition, these are excellent in waterproofness.

Examples 2-1 to 2-6, 2-12 to 2-13, 2-17, and 2-29 to 2-30 used an anionic polymer as the dispersive polymer D for the colorant, and used any one of the anionic polymer CI, the cationic polymer CII, the betaine polymer CIII and a nonionic polymer singly as the polymer C. From these results, it was confirmed that Example 2-1 using the betaine polymer CIII showed a lowest black density, and then Examples 2-2 to 2-4 using the anionic polymer CI showed a relatively low black density, and these Examples are more excellent in concealing performance and coloring performance. In addition, it was confirmed that Examples 2-5 to 2-6 using the cationic polymer CII-1 are more excellent in waterproofness.

Also from Table 4 and Table 5, it was confirmed that, among Examples 2-7 to 2-11, 2-14 to 2-16, and 2-18 to 2-27 using an anionic polymer or a nonionic polymer as the dispersive polymer D for the colorant and using two kinds as the polymer C as combined, Examples 2-7 to 2-9 using the anionic polymer CI and the betaine polymer CIII as combined showed a lowest black density, and then Examples 2-10 to 2-11 using the cationic polymer CII-1 and the betaine polymer CIII as combined showed a relatively low black density, and these Examples are more excellent in concealing performance and coloring performance. In addition, it was confirmed that Examples 2-14 to 2-16, and 2-18 to 2-22 using the anionic polymer CI and the cationic polymer CII-1 or the cationic silicone polymer CII-2 as combined are more excellent in waterproofness.

Further, from Table 5, it was confirmed that Example 2-28 using three kinds of the anionic polymer CI, the cationic silicone polymer CII-2 and the betaine polymer CIII as the polymer C showed a relatively low black density and is excellent in concealing performance and coloring performance and also excellent in waterproofness

Examples 3-1 to 3-11

(Production of Cosmetic Composition)

According to the formulation shown in Table 6, YUKA FORMER SM-dry or YUKA FORMER SM-dry and the cationic silicone polymer 3 as the polymer C (betaine polymer CIII and cationic silicone polymer CII-2) were dissolved in anhydrous ethanol or anhydrous isopropanol as the solvent A; after confirming that the solution was transparent and free from a floating material and a precipitate, the solvent B and water were added as shown in Table 6; the contents were stirred and homogenized; and the resultant mixture was filtered through a cellulose acetate syringe filter having a pore size of 0.20 pm (by Advantech Co., Ltd.) to give a cosmetic composition Z-1 to Z-11.

The dissolved amount of the polymer C (YUKA FORMER SM-dry and cationic silicone polymer 3) used in Examples 3-8 to 3-9 in 100 g of the solvent B shown in Table 6 was less than 5 g, and the dissolved amount thereof in 100 g of the solvent A (anhydrous ethanol) was 5 g or more.

(Makeup Method (production of cosmetic coating film) and Evaluation of Concealing Performance and Coloring Performance)

According to the same method as in Example 1-1, prints having a cosmetic coating film 3-1 to 3-11 were obtained and the image density thereof was measured to evaluate concealing performance and coloring performance. The results are shown in Table 6.

In evaluation of concealing performance and coloring performance, the magenta density was measured every 5 minutes after spraying with fine liquid drops of ion-exchanged water was stopped, and a time necessary until the value of the magenta density became stable was measured, thereby evaluating the coloration expression speed. On the occasion of measuring the magenta density, the point of time when a difference from the magenta density measured 5 minutes ago became 0.1 or less was considered such that the magenta density became stable, and that time was recorded, whereby the magenta density on that occasion was evaluated. The results are shown in Table 6.

The time until the magenta density becomes stable is shorter, such is more preferred. The case where the time is 30 minutes or shorter is not practically problematic in point of the coloration expression speed.

Table 6

| | | | Example | | | | | | | | | | |
|---|---|---|---|---|---|---|---|---|---|---|---|---|---|
| | | | 3-1 | 3-2 | 3-3 | 3-4 | 3-5 | 3-6 | 3-7 | 3-8 | 3-9 | 3-10 | 3-11 |
| Cosmetic Composition No. | | | Z-1 | Z-2 | Z-3 | Z-4 | Z-5 | Z-6 | Z-7 | Z-8 | Z-9 | Z-10 | Z-11 |
| Composition (parts) | Solvent A | Anhydrous Ethanol (boiling point 78°C, Ra 24) | 62.5 | 60.5 | 58.5 | 53.5 | 48.5 | 43.5 | 38.5 | 58.5 | 58.5 | | |
| | | Anhydrous Isopropanol (boiling point 82°C, Ra 28) | | | | | | | | | | 56.5 | 54.5 |
| | Solvent B | PARLEAM 3 (boiling point 179°C, Ra 45) | 6 | 8 | 10 | 15 | 20 | 25 | 30 | | | 10 | 10 |
| | | KF-96A-1cs (boiling point 153°C, Ra 45) | | | | | | | | 10 | | | |
| | | TMF-1.5 (boiling point 191°C, Ra 45) | | | | | | | | | 10 | | |
| | Polymer C | YUKA FORMER SM-dry | 1.5 | 1.5 | 1.5 | 1.5 | 1.5 | 1.5 | 1.5 | 0.75 | 0.75 | 1.5 | 1.5 |
| | | Cationic Silicone Polymer 3 | | | | | | | | 0.75 | 0.75 | | |
| | Colorant Dispersion | Colorant Dispersion 1 | 30 | 30 | 30 | 30 | 30 | 30 | 30 | 30 | 30 | 30 | 30 |
| | Water | | 0 | 0 | 0 | 0 | 0 | 0 | 0 | 0 | 0 | 2 | 4 |
| Ratio by mass of content of solvent A to content of solvent B in cosmetic composition [solvent A/solvent B] | | | 10.4 | 7.6 | 5.9 | 3.6 | 2.4 | 1.7 | 1.3 | 5.9 | 5.9 | 5.7 | 5.5 |
| Viscosity of Cosmetic Composition (mPa·s) | | | 3.6 | 3.7 | 3.8 | 3.8 | 3.8 | 3.9 | 4.0 | 4.5 | 4.6 | 4.1 | 4.3 |
| Evaluation | | Cosmetic Coating Film No. | 3-1 | 3-2 | 3-3 | 3-4 | 3-5 | 3-6 | 3-7 | 3-8 | 3-9 | 3-10 | 3-11 |
| | | Coloration Expression Speed [time until stable coloration (min)] | 10 | 15 | 15 | 15 | 20 | 20 | 20 | 20 | 20 | 15 | 15 |
| | | Magenta Density | 1.70 | 1.70 | 1.70 | 1.71 | 1.71 | 1.71 | 1.71 | 1.68 | 1.67 | 1.65 | 1.64 |
| | | Black Density | 0.67 | 0.60 | 0.53 | 0.46 | 0.40 | 0.48 | 0.54 | 0.78 | 0.82 | 0.79 | 0.83 |
| | | Density Difference (magenta density – black density) | 1.03 | 1.10 | 1.17 | 1.25 | 1.31 | 1.23 | 1.17 | 0.90 | 0.85 | 0.86 | 0.81 |

From Table 6, it was confirmed that Examples 3-1 to 3-11 took a time of coloration expression speed of shorter than 30 minutes, had a low black density and were excellent in concealing performance, had a high magenta density, had a density difference between the magenta density and the black density of more than 0.8 and were excellent in coloration performance.

Industrial Applicability

The cosmetic composition of the present invention is excellent in concealing performance and coloring performance even though not using an inorganic pigment, and can form a good cosmetic coating film applicable to skin, hair or nails.

## Claims

**1.** A cosmetic composition comprising a solvent A, a solvent B, a polymer C and a colorant, wherein:

the solvent A is at least one selected from the group consisting of ethanol, n-propanol and isopropanol,

the boiling point of the solvent B is 150°C or higher, and the distance Ra of the Hansen solubility parameter of the solvent B to water as expressed by the following equation (1) is 40 or more,

the solvent B is compatible with the solvent A, and the polymer C is soluble in the solvent A and insoluble in the solvent B:

$$Ra=(4\times\Delta D^2+\Delta P^2+\Delta H^2)^{0.5} \qquad (1)$$

wherein,

$\Delta D$ is a difference of dispersing component in the Hansen solubility parameter between the solvent B and water,
$\Delta P$ is a difference of polar component in the Hansen solubility parameter between the solvent B and water, and
$\Delta H$ is a difference of hydrogen-bonding component in the Hansen solubility parameter between the solvent B and water.

**2.** The cosmetic composition according to claim 1, wherein the colorant is dispersed with a dispersive polymer D.

**3.** The cosmetic composition according to claim 2, wherein the dispersive polymer D is soluble in the solvent A and is insoluble in the solvent B.

**4.** The cosmetic composition according to any one of claims 1 to 3, wherein the polymer C comprises a polymer that comprises, as a structural unit, at least one selected from the group consisting of an acid group-containing monomer, a basic group-containing monomer and a betaine monomer.

**5.** The cosmetic composition according to any one of claims 1 to 4, wherein the solvent B is at least one selected from the group consisting of a volatile hydrocarbon oil and a volatile silicone oil.

**6.** The cosmetic composition according to any one of claims 1 to 5, wherein a content of water is 5% by mass or less.

**7.** The cosmetic composition according to any one of claims 1 to 6, wherein a content of the solvent A is 30% by mass or more and 90% by mass or less.

**8.** The cosmetic composition according to any one of claims 1 to 7, wherein a content of the solvent B is 5% by mass or more and 40% by mass or less.

**9.** The cosmetic composition according to any one of claims 1 to 8, wherein a content of the polymer C is 0.5% by mass or more and 15% by mass or less.

**10.** The cosmetic composition according to any one of claims 1 to 9, wherein a content of the colorant is 1% by mass or more and 15% by mass or less.

**11.** The cosmetic composition according to any one of claims 1 to 10, which is a cosmetic composition for hair.

**12.** A makeup method using the cosmetic composition of any one of claims 1 to 10, which comprises:

Step 1: a step of applying the cosmetic composition to skin, hair or nails, and
Step 2: a step of applying liquid drops of a water-containing liquid E to the cosmetic composition applied on skin, hair or nails.

**13.** The makeup method according to claim 12, wherein a content of water in the liquid E is 50% by mass or more.

**14.** The makeup method according to claim 12 or 13, wherein the application method for the cosmetic composition in the step 1 is a method of ejecting the cosmetic composition according to at least one selected from the group consisting of an inkjet method and a dispenser method to apply liquid drops of the cosmetic composition to skin, hair or nails.

**15.** The makeup method according to any one of claims 12 to 14, wherein the application method for the liquid drops in the step 2 is a method of spraying liquid drops using an atomizing device, or a method of ejecting liquid drops according to an inkjet method.

**16.** A makeup method using a cosmetic composition, wherein:

the cosmetic composition comprises a solvent A, a solvent B, a polymer C and a colorant,
the solvent A is at least one selected from the group consisting of ethanol, n-propanol and isopropanol,
the boiling point of the solvent B is 150°C or higher, and the distance Ra of the Hansen solubility parameter of the solvent B to water as expressed by the following equation (1) is 40 or more,
the solvent B is compatible with the solvent A, and the polymer C is soluble in the solvent A and insoluble in the solvent B,
the makeup method comprises:

Step 1: a step of ejecting the cosmetic composition according to at least one selected from the group consisting of an inkjet method or a dispenser method to apply liquid drops of the cosmetic composition to skin, hair or nails, and
Step 2: a step of applying liquid drops of a water-containing liquid E to the cosmetic composition applied on skin, hair or nails,
the application method for liquid drops in the step 2 is a method of spraying liquid drops using an atomizing device, or ejecting liquid drops according to an inkjet method,

$$Ra=(4\times\Delta D^2+\Delta P^2+\Delta H^2)^{0.5} \qquad (1)$$

wherein,

$\Delta D$ is a difference of dispersing component in the Hansen solubility parameter between the solvent B and water,
$\Delta P$ is a difference of polar component in the Hansen solubility parameter between the solvent B and water, and
$\Delta H$ is a difference of hydrogen-bonding component in the Hansen solubility parameter between the solvent B and water.

**17.** A cosmetic coating film formed from the cosmetic composition of any one of claims 1 to 11.

**INTERNATIONAL SEARCH REPORT**

International application No. PCT/JP2021/021213

**A. CLASSIFICATION OF SUBJECT MATTER**

Int.Cl. A61K8/34(2006.01)i, A61K8/31(2006.01)i, A61K8/49(2006.01)i, A61K8/81(2006.01)i, A61K8/891(2006.01)i, A61K8/898(2006.01)i, A61Q1/02(2006.01)i, A61Q3/00(2006.01)i, A61Q5/00(2006.01)i
FI: A61K8/34, A61K8/31, A61K8/49, A61K8/81, A61K8/891, A61K8/898, A61Q1/02, A61Q3/00, A61Q5/00

According to International Patent Classification (IPC) or to both national classification and IPC

**B. FIELDS SEARCHED**

Minimum documentation searched (classification system followed by classification symbols)
Int.Cl. A61K8/34, A61K8/31, A61K8/49, A61K8/81, A61K8/891, A61K8/898, A61Q1/02, A61Q3/00, A61Q5/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2018-058775 A (ARIMINO KAGAKU KK) 12 April 2018 (2018-04-12), example 1 | 1-11, 17<br>12-16 |
| Y | JP 2004-269514 A (OZAKI, Michi) 30 September 2004 (2004-09-30), claims, examples | 12-16 |
| Y | JP 61-183208 A (KOBAYASHI KOSE COMPANY LIMITED) 15 August 1986 (1986-08-15), claims, examples | 12-16 |
| Y | JP 2020-026401 A (SHISEIDO CO., LTD.) 20 February 2020 (2020-02-20), paragraphs [0005], [0065], [0081] | 12-16 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>04 August 2021 | Date of mailing of the international search report<br>17 August 2021 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No. PCT/JP2021/021213

C (Continuation). DOCUMENTS CONSIDERED TO BE RELEVANT

| Category* | Citation of document, with indication, where appropriate, of the relevant passages | Relevant to claim No. |
|---|---|---|
| X | JP 2017-122076 A (KAO CORPORATION) 13 July 2017 (2017-07-13), formulation example 3 | 1, 4-5, 9-10, 17 |
| A | WO 2019/073743 A1 (PANASONIC INTELLECTUAL PROPERTY MANAGEMENT CO., LTD.) 18 April 2019 (2019-04-18), claims, examples | 1-17 |

Form PCT/ISA/210 (second sheet) (January 2015)

**INTERNATIONAL SEARCH REPORT**
Information on patent family members

International application No.
PCT/JP2021/021213

| | | |
|---|---|---|
| JP 2018-058775 A | 12 April 2018 | (Family: none) |
| JP 2004-269514 A | 30 September 2004 | (Family: none) |
| JP 61-183208 A | 15 August 1986 | (Family: none) |
| JP 2020-026401 A | 20 February 2020 | WO 2020/031921 A1 |
| JP 2017-122076 A | 13 July 2017 | (Family: none) |
| WO 2019/073743 A1 | 18 April 2019 | US 2020/0216693 A1<br>claims, examples<br>EP 3695828 A1<br>CN 111182882 A<br>SG 11202002896S A |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016121137 A
- JP 2015520120 A
- JP 2011126978 A


### Non-patent literature cited in the description


- HANSEN SOLUBILITY PARAMETERS. A User's Handbook